(19) Europäisches Patentamt
European Patent Office
Office européen des brevets

(11) **EP 4 118 652 B1**

(12) **EUROPEAN PATENT SPECIFICATION**

(45) Date of publication and mention
of the grant of the patent:
**06.05.2026  Bulletin 2026/19**

(21) Application number: **21709709.6**

(22) Date of filing: **09.03.2021**

(51) International Patent Classification (IPC):
**G16B 20/10** (2019.01)    **G16B 20/20** (2019.01)
**G16B 20/40** (2019.01)

(52) Cooperative Patent Classification (CPC):
**G16B 20/10; G16B 20/20; G16B 20/40**

(86) International application number:
**PCT/EP2021/055920**

(87) International publication number:
**WO 2021/180722 (16.09.2021 Gazette 2021/37)**

(54) **METHOD FOR THE ANALYSIS OF GENETIC MATERIAL**

VERFAHREN ZUR ANALYSE VON GENETISCHEM MATERIAL

PROCÉDÉ POUR L'ANALYSE DE MATÉRIAU GÉNÉTIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.03.2020  EP 20162599**

(43) Date of publication of application:
**18.01.2023  Bulletin 2023/03**

(73) Proprietor: **Vrije Universiteit Brussel
1050 Brussel (BE)**

(72) Inventor: **VERDYCK, Pieter
1090 Brussel (BE)**

(74) Representative: **Arnold & Siedsma
Bezuidenhoutseweg 57
2594 AC The Hague (NL)**

(56) References cited:
WO-A1-2015/028576    WO-A1-2020/016477
WO-A2-2007/075836    US-A1- 2008 318 235
US-A1- 2011 288 780    US-A1- 2012 196 754
US-A1- 2014 287 934

• MARIN D ET AL: "Validation of a targeted next generation sequencing-based comprehensive chromosome screening platform for detection of triploidy in human blastocysts", REPRODUCTIVE BIOMEDICINE ONLINE, ELSEVIER, AMSTERDAM, NL, vol. 36, no. 4, 2 January 2018 (2018-01-02), pages 388 - 395, XP085369845, ISSN: 1472-6483, DOI: 10.1016/J.RBMO.2017.12.015

**Description**

FIELD OF THE INVENTION

The present invention is directed to a method for the analysis of genetic material in a subject. More in particular, the present invention relates to a method for the analysis of genetic material using unphased genotype information of polymorphic variants of a first and second parent of a subject in combination with the allele frequency of the polymorphic variants in the genetic material of the subject. In a further aspect, the method of the present invention is particularly useful for the analysis of genetic material isolated from a sample comprising a low amount of genetic material and/or detection of low level chromosomal mosaicism.

BACKGROUND TO THE INVENTION

Chromosome anomalies include a wide variety of anomalies such as errors in ploidy, aneuploidy, structural chromosomal rearrangements and uniparental disomy (UPD). Errors in ploidy are defined by the presence of an abnormal number of complete chromosome sets. In human and mammals this corresponds to the presence of only one (monoploidy or haploidy) or more than two (3: triploidy, 4: tetraploidy, >2: polyploidy) complete sets of chromosomes instead of two (diploidy) in somatic cells. Aneuploidy refers to chromosomal abnormalities in which too few or too many copies of one or more chromosomes are observed (nullisomy, monosomy, trisomy, tetrasomy, etc.). Structural chromosomal rearrangements are abnormalities in which the structure of one or more chromosomes is altered. These include reciprocal translocations, Robertsonian translocations, deletions, duplications, insertions, inversions, etc.. Structural chromosomal rearrangements can be either (apparently) balanced (no loss or gain of genetic material) or unbalanced (loss or gain of genetic material). Last, uniparental disomy (UPD) is caused by the inheritance of two chromosome copies from the same parent which, depending on the chromosome at hand and the parental origin of the chromosomes, can cause disease.

Chromosomal anomalies are very important in medicine as they cause a multitude of disorders and syndromes (Schinzel 2001). In addition, they play a role in infertility and miscarriage. To date many techniques have been developed to detect chromosomal anomalies. They include karyotyping, Fluorescence In Situ Hybridization (FISH), array comparative genome hybridization (aCGH), single nucleotide polymorphism (SNP) array, (quantitative-) polymerase chain reaction (PCR) and more recently sequencing based methods. These methods all have their own strengths and weaknesses but generally work well on relative large amounts of genomic DNA or a large amount of cells. Diagnosis is much more difficult when analyzing DNA obtained from only few cells. In this case amplification methods are required prior to molecular analysis which introduces a bias in locus- or allelic representation. By chance a given locus can be over- or underrepresented after amplification. In addition, one allele can be overrepresented compared to the other(s). These phenomena cause the data to be more noisy and interpretation is more difficult. Also chromosomal mosaicism, a phenomenon in which not all analyzed cells are chromosomally identical, adds to the complexity and can make interpretation more difficult. We created a new method for improved detection of chromosomal anomalies using data generated by high throughput genotyping technologies such as SNP array and sequencing.

When high throughput genotyping technologies are used for detection of chromosomal anomalies such as SNP array or sequencing, the sample of the subject is mostly analyzed by itself without the use of genotype data from both parents of the subject. By taking into account the genotype data from both parents and using the allele frequency values obtained in the sample of the subject, the methods described in the current invention allow a more accurate detection of aneuploidy in samples with low amounts of target DNA and improve the detection of chromosomal mosaicism. In addition, in some embodiments of the invention, the method allows discrimination between meiotic and mitotic chromosome anomalies.

In the present invention, methods have been identified for the analysis of genetic material of a subject without the need of phased genotype data. In particular, typical for the present invention is that only unphased genotype information of polymorphic variants of a first and a second parent of the subject is needed. As a result, the present invention thus allows using a DNA or cell sample from both parents and subject only, without a phasing reference sample. This is different from other SNP based methods which rely on haplotyping such as siCHILD (e.g. WO2015028576)(Zamani Esteki et al. 2015) and karyomapping (Handyside et al. 2010, Natesan et al. 2014). In the methods already known in the prior art, the use of a DNA sample from a closely related family member is required from each parent to perform reliable aneuploidy detection. This is typically a sibling or a maternal and a paternal grandparent. These samples are a burden to come by as the parents do not always wish to disclose the need for preimplantation genetic testing and/or in-vitro fertilization to both sides of the family. In other cases, the (prospective) grandparents are deceased or contact has been lost. Also in situations where preimplantantation genetic testing is offered with SNP array or sequencing with the use of one grandparental reference (e.g. maternal side), aneuploidy can now be detected without the need to ask a sample from a grandparent from the other parent's side (e.g. paternal side). In addition, it greatly improves aneuploidy detection when performing karyomapping which is currently neither intended nor validated for aneuploidy detection.

## SUMMARY OF THE INVENTION

The present invention is related to methods for the analysis of genetic material in a subject. In particular, a method for the analysis of genetic material in a subject is disclosed, wherein said method comprises:

- obtaining unphased genotype information of polymorphic variants of a first and second parent of the subject;
- obtaining the genomic location of the polymorphic variants;
- selection of the polymorphic variants based on the following criteria:

  ◦ polymorphic variants for which the first and second parent are homozygous or hemizygous for a different allele (herein also called the category 1 polymorphic variants);
  ◦ polymorphic variants for which the first parent is homozygous or hemizygous for a specific allele and the second parent is heterozygous for said specific allele (herein also called the category 2 polymorphic variants);
  ◦ polymorphic variants for which the second parent is homozygous or hemizygous for a specific allele and the first parent is heterozygous for said specific allele (herein also called the category 3 polymorphic variants);

- obtaining the allele frequency (AF) values for said selected polymorphic variants in genetic material of the subject;
- selection of one allele per polymorphic variant and subcategorization of its corresponding AF frequency of the subject in one of the following subcategories:

  ◦ AF values of the category 1 polymorphic variants, representing the AF values for alleles present in homozygous or hemizygous state in the first parent (subcategory 1A polymorphic variants);
  ◦ AF values of the category 1 polymorphic variants, representing the AF values for alleles present in homozygous or hemizygous state in the second parent (subcategory 1B polymorphic variants);
  ◦ AF values of the category 2 polymorphic variants, representing the AF values for alleles present in homozygous or hemizygous state in the first parent (subcategory 2A polymorphic variants);
  ◦ AF values of the category 2 polymorphic variants, representing the AF values for alleles heterozygous in the second parent and absent in the first parent (subcategory 2B polymorphic variants);
  ◦ AF values of the category 3 polymorphic variants, representing the AF values for alleles present in homozygous or hemizygous state in the second parent (subcategory 3A polymorphic variants);
  ◦ AF values of the category 3 polymorphic variants, representing the AF values for alleles heterozygous in the first parent and absent in the second parent (subcategory 3B polymorphic variants);

- evaluation whether a genetic anomaly is present in the genetic material of the subject based on the AF values of the polymorphic variants in one or more of the subcategories and the genomic location of said polymorphic variants.

In a further embodiment, the method of the present invention further comprises calculation of the mean AF values, the trimmed, or also called truncated, mean AF values or the median AF values of the polymorphic variants for a given subcategory, wherein the polymorphic variants are located between two genomic locations on a chromosome, and evaluation whether a genetic anomaly is present in the genetic material of the subject based on said mean, trimmed or truncated mean or median AF values of polymorphic variants observed between said genomic locations directly, or optionally by calculating the difference between the mean AF values, the trimmed mean AF values or median AF values of the given subcategory and said difference being indicated as "delta AF", and followed by evaluation whether a genetic anomaly is present in the genetic material of the subject based on the "delta AF" values observed between said genomic locations

In a specific embodiment, the present invention discloses a method for the analysis of genetic material in a subject, said method comprising:

- obtaining unphased genotype information of polymorphic variants of a first and second parent of the subject;
- obtaining the genomic location of the polymorphic variants;
- selection of the polymorphic variants for which the first and second parent are homozygous or hemizygous for a different allele (also referred herein as category 1 polymorphic variants);
- obtaining the allele frequency (AF) values for the selected category 1 polymorphic variants in the genetic material of the subject;
- selection of one allele per polymorphic variant and subcategorization of its corresponding AF frequency of the subject in one of the following subcategories:

  ◦ AF values representing the AF values for alleles present in homozygous or hemizygous state in the first parent (subcategory 1A polymorphic variants);
  ◦ AF values representing the AF values for alleles present in homozygous or hemizygous state in the second parent (subcategory 1B polymorphic variants);

- evaluation whether a genetic anomaly is present in the genetic material of the subject based on the AF values of the subcategory 1A or 1B polymorphic variants and the genomic location of said polymorphic variants.

In a further embodiment, said method comprises calculation of the mean AF values, the trimmed mean AF values or the median AF values of the polymorphic variants for a given subcategory 1A or 1B, wherein the polymorphic variants are located between two particular genomic locations on a chromosome, and evaluating whether a genetic anomaly is present in the genetic material of the subject based on said mean, trimmed mean or median AF values of polymorphic variants observed between said genomic locations directly, or optionally by calculating the difference between the mean AF values, the trimmed mean AF values or median AF values of subcategories 1A and 1B and said difference being indicated as "delta AF", and followed by evaluation whether a genetic anomaly is present in the genetic material of the subject based on the "delta AF" values observed between said genomic locations. In still a further embodiment, the AF values, mean AF values, trimmed mean AF values or delta AF values are used to calculate a value for the parental contribution between said genomic locations and evaluating whether a genetic anomaly is present in the genetic material of the subject based on said value for parental contribution observed between said genomic locations. In still a further embodiment, the AF values, mean AF values, trimmed mean AF values, delta AF values or value for parental contribution between said genomic locations and the detected copy number between said genomic locations is used to calculate the copy number originating from parent 1 and the copy number originating from parent 2 between said genomic locations and evaluating whether a genetic anomaly is present in the genetic material of the subject based on said copy numbers originating from parent 1 and parent 2 observed between said genomic locations.

In another specific embodiment, the present invention discloses a method for the analysis of genetic material in a subject, said method comprising:

- obtaining unphased genotype information of polymorphic variants of a first and second parent of the subject;
- obtaining the genomic location of the polymorphic variants;
- selection of the polymorphic variants for which the first parent is homozygous or hemizygous for a specific allele and the second parent is heterozygous for said specific allele (also referred herein as category 2 polymorphic variants);
- obtaining the allele frequency (AF) values for the selected category 2 polymorphic variants;
- selection of one allele per polymorphic variant and subcategorization of its corresponding AF frequency of the subject in one of the following subcategories:

  ◦ AF values representing the AF values for alleles present in homozygous or hemizygous state in the first parent (subcategory 2A polymorphic variants);
  ◦ AF values representing the AF values for alleles heterozygous in the second parent and absent in the first parent (subcategory 2B polymorphic variants);

- evaluation whether a genetic anomaly is present in the genetic material of the subject based on the AF values of the subcategory 2A or 2B polymorphic variants and the genomic location of said polymorphic variants.

In a further embodiment, said method comprises calculation of the mean AF values, the trimmed mean AF values or the median AF values of the polymorphic variants for a given subcategory 2A or 2B, wherein the polymorphic variants are located between two particular genomic locations on a chromosome, and evaluating whether a genetic anomaly is present in the genetic material of the subject based on said mean, trimmed mean or median AF values of polymorphic variants observed between said genomic locations directly, or optionally by calculating, the difference between the median AF values, the mean AF values or the trimmed mean AF values of subcategories 2A and 2B and said difference being indicated as "delta AF", and followed by evaluation whether a genetic anomaly is present in the genetic material of the subject based on the "delta AF" values observed between said genomic locations.

In still a further embodiment, the AF values, mean AF values, trimmed mean AF values, delta AF values or value for parental contribution between said genomic locations and the detected copy number between said genomic locations is used to calculate the copy number originating from parent 1 and the copy number originating from parent 2 between said genomic locations and evaluating whether a genetic anomaly is present in the genetic material of the subject based on said copy numbers originating from parent 1 and parent 2 observed between said genomic locations.

In still another embodiment, the present invention discloses a method for the analysis of genetic material in a subject, said method comprising:

- obtaining unphased genotype information of polymorphic variants of a first and second parent of the subject;
- obtaining the genomic location of the polymorphic variants;
- selection of the polymorphic variants for which the second parent is homozygous or hemizygous for a specific allele and the first parent is heterozygous for said specific allele (also referred herein as category 3 polymorphic variants);
- obtaining the allele frequency (AF) values for the selected category 3 polymorphic variants;
- selection of one allele per polymorphic variant and subcategorization of its corresponding AF frequency of the subject in one of the following subcategories:

  ∘ AF values representing the AF values for alleles present in homozygous or hemizygous state in the second parent (subcategory 3A polymorphic variants);
  ∘ AF values representing the AF values for alleles heterozygous in the first parent and absent in the second parent (subcategory 3B polymorphic variants);

- evaluation whether a genetic anomaly is present in the genetic material of the subject based on the AF values of the subcategory 3A or 3B polymorphic variants and the genomic location of said polymorphic variants.

In a further embodiment, said method comprises calculation of the mean AF values, the trimmed mean AF values or the median AF values of the polymorphic variants for a given subcategory 3A or 3B, wherein the polymorphic variants are located between two particular genomic locations on a chromosome, and evaluating whether a genetic anomaly is present in the genetic material of the subject based on said mean or media AF values of polymorphic variants observed between said genomic locations directly, or optionally by calculating the difference between the median AF values, the mean AF values, or the trimmed mean AF values of subcategories 3A and 3B and said difference being indicated as "delta AF", and followed by evaluation whether a genetic anomaly is present in the genetic material of the subject based on the "delta AF" values observed between said genomic locations.

In a further embodiment, the method according to any of the embodiments, further comprises selection of the AF values using an upper and lower cut-off value. In a more specific embodiment, the selection of the AF values is performed for subcategory2A, 2B, 3A and/or 3B polymorphic variants. Even more in particular, the selection of the AF values using an upper and lower cut-off value is essential for subcategory 2A, 2B, 3A and/or 3B polymorphic variants. In still a further embodiment, the selection of the AF values is performed using fixed upper and lower cut-off values. In other words, AF values are selected when they are above or below a specifically defined cut-off value. In another embodiment, the selection of the AF values is performed using variable upper and lower cut-off values.

In a further embodiment, the method according to any of the embodiments, further comprises excluding the AF values for polymorphic variants located within detected both parental homolog (BPH) segments. In a further specific embodiment, excluding the AF values for polymorphic variants located within detected both parent homolog (BPH) segments is performed for subcategory 2A, 2B, 3A, 3B polymorphic variants only, and not for category 1 polymorphic variant. In a more specific embodiment AF values are excluded for subcategory 2A and 2B polymorphic variants within a BPH segment if the said BPH segment is inherited from parent 2 and AF values are excluded for subcategory 3A and 3B polymorphic variants within a BPH segment if the said BPH segment is inherited from parent 1.

Within the methods according to the invention the evaluation whether a genetic anomaly is present in the genetic material of the subject can be based on the mean AF values, the trimmed mean AF values or the median AF values of the polymorphic variants for a given subcategory. In said instances a genetic anomaly is present when the mean AF values, the trimmed mean AF values or the median AF values deviates from 0.5; in particular when the AF value deviates from 0.5 with a value of at least and about 0.025 ; more in particular when the AF value deviates from 0.5 with a value of at least and about 0.045.

In a particular embodiment, a deviation of the mean AF value, the trimmed mean AF value or the median AF value from 0.5 with a value up to and about 0.045 (in particular up to and about 0.0462) is considered normal (normal disomy); when the mean, trimmed mean or median AF value deviates from 0.5 above a threshold of about 0.1 (in particular above 0.117) and an increased copynumber is observed, the value indicates a full trisomy; and when the mean, trimmed mean or median AF value deviates from 0.5, with a value between and about 0.045 to about 0.1 ; in particular between and about 0.0462 to about 0.117; and an increased copynumber is observed,the sample is categorized as mosaic trisomy/disomy. When the mean, trimmed mean or median AF value deviates from 0.5 above a threshold of about 0.45 and a decreased copynumber is observed, the value indicates a full monosomy; and when the mean, trimmed mean or median AF value deviates from 0.5, with a value between and about 0.045 to about 0.45 ; in particular between and about 0.0462 to about 0.45; and a decreased copynumber is observed,the sample is categorized as mosaic monosomy/disomy.

Optionally, within the methods according to the invention the evaluation whether a genetic anomaly is present in the genetic material of the subject can be based on the "delta AF" values observed between said genomic locations wherein. In said instances a genetic anomaly is present when the delta AF value deviates from 0; in particular when the delta AF value deviates from 0 with a value of at least and about 0.05; more in particular when the delta AF value deviates from 0 with a value of at least and about 0.09.

In a particular embodiment, a deviation of the delta AF value below the threshold of about 0.09 (in particular below 0.0924) is considered normal (normal disomy); when the delta AF value deviates from 0 above a threshold of about 0.2 (in particular above 0.234) and an increased copynumber is observed, the value indicates a full trisomy or duplication; and when the delta AF value deviates from 0, between both threshold values ) and an increased copynumber is observed the sample is categorized as mosaic disomy/trisomy or mosaic duplication); when the delta AF value deviates from 0 above a threshold of about 0.9 and a decreased copynumber is observed, the value indicates a full monosomy or deletion; and when the delta AF value deviates from 0, between both threshold values and an decreased copynumber is observed the sample is categorized as mosaic monosomy/disomy or mosaic deletion.

In a further aspect, and in all methods according to the different embodiments of the present invention, the AF values, the mean AF values, the trimmed mean AF values, the median AF values or the delta AF values are visualized per subcategory of polymorphic variants.

In still a further embodiment, the AF values, mean AF values, trimmed mean AF values or delta AF values are used to calculate a value for the parental contribution between said genomic locations and evaluating whether a genetic anomaly is present in the genetic material of the subject based on said value for parental contribution observed between said genomic locations.

Within the methods according to the invention, a value for the parental contribution between said genomic locations, and in particular a value for the percentage maternal contribution (%Mat) or a value for the percentage paternal contribution (%Pat) (wherein %Pat = 100 - %Mat) , is based on a second order generalized linear model between the delta AF values and the percentage parental contribution, i.e. %Mat or %Pat, across said genomic locations; and wherein a paternal contribution deviating from 50%; in particular a deviation of at least and about 3%; more in particular a deviation of at least and about 6%; is indicative for a chromosomal anomaly.

In a particular embodiment a %Mat or %Pat between and about 44.4% and 55.6% is indicative for a normal disomy; wherein a %Mat or %Pat between and about 63.6% and 72.7% is indicative for a 'both parental homolog' (BPH) trisomy; and wherein a %Mat or %Pat between and about 0% and 3.3%, is indicative for autosome monosomies.

In another particular embodiment a value for the percentage maternal contribution (%Mat) is based on a second order generalized linear model between the delta AF values and the maternal contribution, across said genomic locations; and wherein a percentage maternal contribution deviating from 50%; in particular a deviation of at least and about 3%; more in particular a deviation of at least and about 6%; is indicative for a chromosomal anomaly.

In a particular embodiment a %Mat between and about 44.4% and 55.6% is indicative for a normal disomy; wherein a %Mat between and about 63.6% and 72.7% is indicative for a complete (non mosaic) trisomy of maternal origin; ; wherein a %Pat between and about 63.6% and 72.7% is indicative for a complete (non mosaic) trisomy of paternal origin; wherein a %Mat between and about 0% and 3.3%, is indicative for a monosomy of maternal origin. and where a %Pat between and about 0% and 3.3%, is indicative for a monosomy of paternal origin.

In another aspect, and in all methods according to the different embodiments of the present invention, the selected allele per polymorphic variant is an allele with a specific feature. In particular, said specific feature is selected from the A allele, the B allele, the allele with the higher allele frequency in a given population, the allele with the lower allele frequency in a given population, the reference allele in a given reference genome, the allele present in homozygous state in parent 1, the allele present in homozygous state in parent 2, the allele present in heterozygous state in parent 1 but absent in parent 2 or the allele present in heterozygous state in parent 2 but absent in parent 1. In an even more preferred embodiment, the selected allele per polymorphic variant is the B allele. In still an even more preferred embodiment, the selected allele per polymorphic variant is the B allele comprising a single nucleotide polymorphism (SNP).

In a specific aspect, in the method of the present invention the selected AF values of the polymorphic variants are converted into discrete genotype calls and it is evaluated whether homozygous or heterozygous AF values are underrepresented or overrepresented between two particular genomic locations. In still a further embodiment, said selected AF values are the selected AF values of the polymorphic variants of the subcategories 2A, 2B, 3A and/or 3B and said AF values are converted into discrete genotype calls and it is evaluated whether homozygous or heterozygous AF values are underrepresented or overrepresented between two particular genomic locations.

The method according to all the different embodiments of the present invention can be combined with a method of haplotyping, such as SiCHILD or karyomapping and/or a method for detection of (relative) DNA quantity such as SNP array, array CGH or sequencing; preferably SNParray and karyomapping. This in a particular embodiment, the present invention discloses a method for the analysis of genetic material in a subject, said method comprising a method of any of the herein above described embodiments in combination with a method of haplotyping and/or a method for the detection of DNA quantity. In a specific embodiment, a method for the analysis of genetic material in a subject is disclosed, said method comprising a method of any of the herein above described embodiments in combination with a method of haplotyping which is karyomapping and/or a method for the detection of DNA quantity which is a SNParray.

The methods according to the different embodiments of this invention comprise obtaining the genomic location of the polymorphic variants and calculating a value for selected alleles of selected variants located between two genomic locations. In a specific embodiment, said genomic locations are the start and end of a chromosome. In another specific embodiment, said genomic locations are the start and end of a chromosome arm. In another specific embodiment said locations are the start and end of a chromosome band. In another specific embodiment the said locations are separated with a fixed distance. In another specific embodiment the locations are determined using a a segmentation algorithm such as a sliding window approach or using spline fitting. Preferably cubic smoothing spline fitting is used to identify the appropriate window sizes.

The methods according to the present invention are thus for the analysis of genetic material in a subject. In a preferred embodiment, said genetic material is genomic DNA.

In another aspect, the genetic material of the subject that is analysed in the methods of the present invention can be isolated from a sample comprising a low amount of genetic material of the subject. In a preferred embodiment, the genetic material of the subject that is analyzed in the methods of the present invention is isolated from a sample comprising a low amount of genetic material of the subject. Preferably, the sample comprises only one, two or a few cells of said subject. In another embodiment, the sample comprising the genetic material is a plasma sample obtained from a mother being pregnant with the subject. In another aspect, the sample comprising the genetic material is genomic DNA from the subject and the analysis aims at detection low level mosaicism. In said aspect, a high amount of genetic material is present in the sample.

As already outlined above, the methods according to the different embodiments of the present invention are for evaluation whether a genetic anomaly is present in the genetic material of the subject. In one embodiment, said genetic anomaly is a numerical or structural chromosomal anomaly. In particular, the genetic anomaly is a numerical or structural chromosomal anomaly selected from a monosomy, uniparental disomy, trisomy, tetrasomy, a tandem duplication, a deletion and combinations thereof. If such a numerical or structural chromosomal anomaly is present in all of the genetic material of the subject, e.g. in all of the biopsied cells, the genetic anomay is a non-mosaic genetic anomaly. In case said numerical or structural chromosomal anomaly is present in part of the genetic material of the subject, e.g. in part of the biopsied cells, the genetic anomaly is a mosaic genetic anomaly. In another embodiment, said genetic anomaly is a mosaic numerical or structural chromosomal anomaly. In particular, the genetic anomaly is a mosaic numerical or structural chromosomal anomaly selected from a mosaic monosomy, mosaic disomy, mosaic trisomy, mosaic tetrasomy, a mosaic tandem duplication, a mosaic deletion and combinations thereof.

In a further aspect, the polymorphic variants that are obtained or selected in the methods of the present invention are selected from single nucleotide polymorphisms (SNPs), short tandem repeats (STRs); preferably the polymorphic variants are SNPs.

The present invention is further also directed to a report displaying the AF values, the mean AF values, the trimmed mean AF values or, the median AF values or the delta AF values obtainable by the methods according to any of the embodiments of the present invention.

In a further embodiment, the data input will be used to a machine-learning algorithm, which could learn to recognize different patterns from a large collection of labeled training samples. Different types of input features will be tested, including the raw data but also features derived from the data. The rules that will determine the final call can eventually also be learned from then training data, using algorithms such as random forests or gradient boosting and/or ensemble strategies. In still a further embodiment, machine learning can be used.

Yet in another aspect, also a computer program which is capable, when executed on a processing engine, to perform the methods according to any of the disclosed embodiments, is disclosed.

The present invention further also discloses a non-transitory machine-readable storage medium storing said computer program.

In another aspect, the present invention discloses a non-transitory machine-readable storage medium storing the AF values, the mean AF values, the trimmed mean AF values, the median AF values or the delta AF values obtained by the methods of the present invention.

In a final aspect, the present invention further discloses a graphical user interface adapted for use of the method of the present invention.

BRIEF DESCRIPTION OF THE DRAWINGS

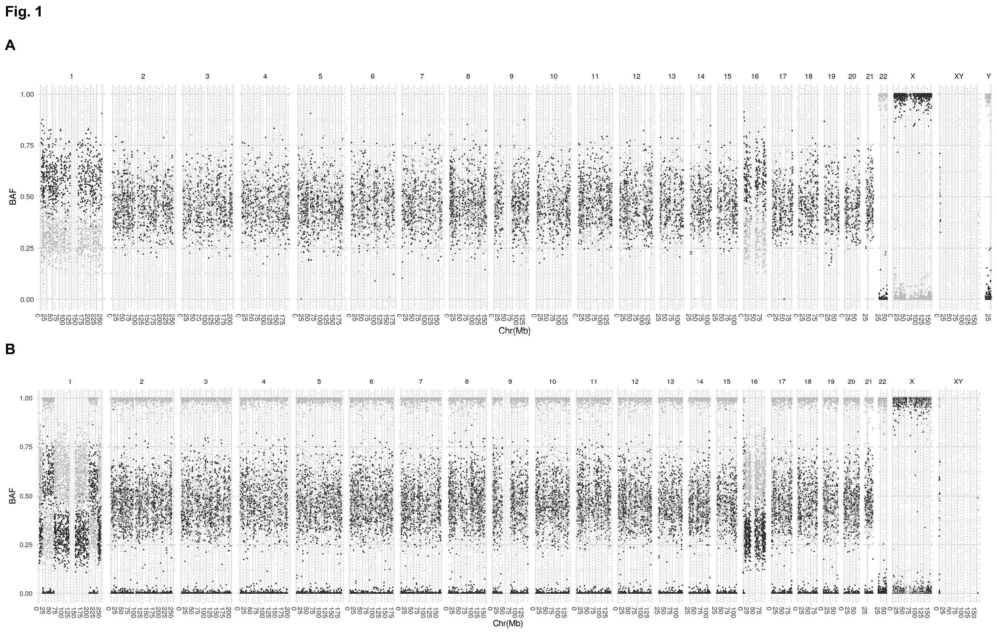

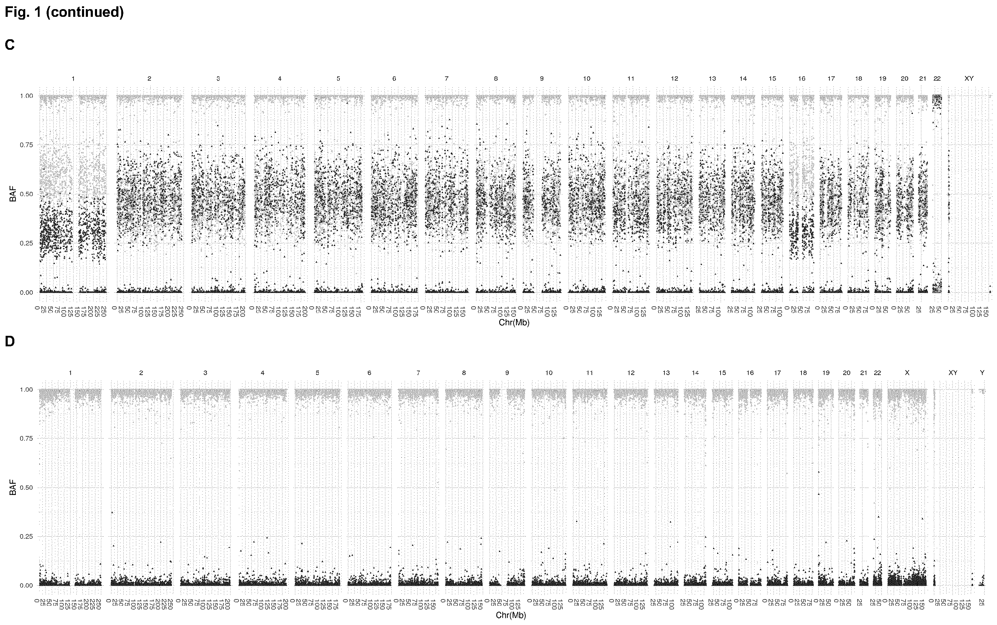

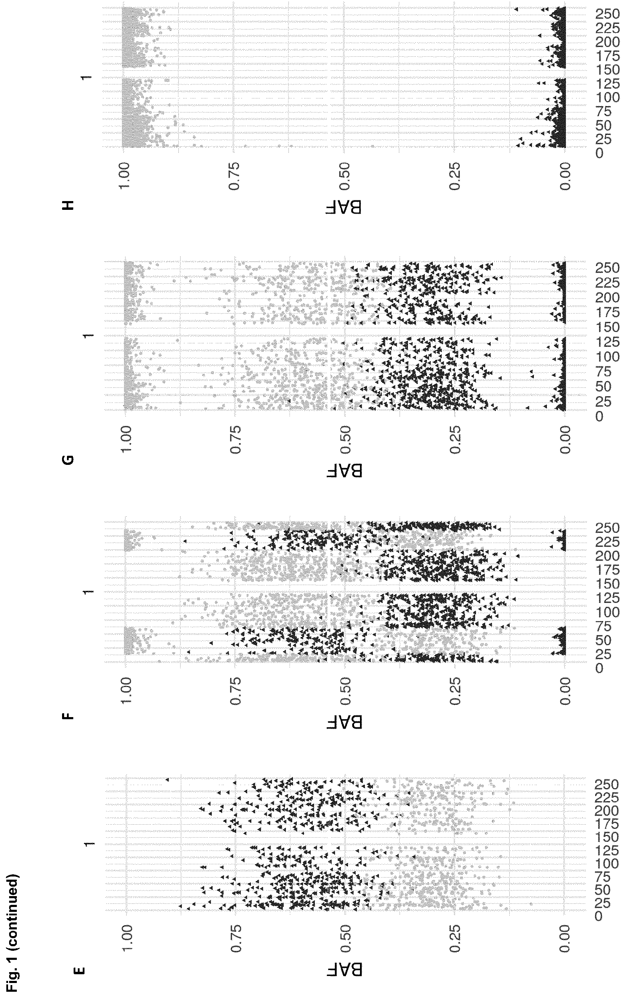

**Figure 1.** Results from an embryo with a 47,XY,+1,+16,-22 molecular karyotype are shown (MDA amplified trophectoderm biopsy obtained at day six after fertilization). In the absence of a phasing reference, the presence of a maternal monosomy 22 and a maternal meiotic trisomy 1 and 16 can be detected. Panels A, B, C, D, F, G, H: the BAF plotted against the genomic position in Mb. **Panel A:** the BAF of category 1 BAF plotted against the genomic position in Mb. Category 1 SNPs are expected to be heterozygous in a euploid female embryo. These are SNPs for which both parents are homozygous but for a different allele (subcategory 1A in light grey circles: paternal BB and maternal AA genotype; subcategory 1B in dark grey triangles: paternal AA and maternal BB genotype). We also added the SNPs on the Y chromosome to this panel for which a BB (to subcategory 1A) or AA (to subcategory 1B) call was assigned to the paternal sample and a very low signal intensity ($\log_2 R$ <-4) or no call was obtained for the maternal sample. We used these categories of SNPs for further analysis as the BAF of these SNPs reflect the paternal (category 1A) and maternal contribution (category 1B). **Panel B:** BAF of category 2 SNPs: maternal informative SNPs. Subcategory 2A (paternal BB and maternal AB call) is shown in light grey circles and subcategory 2B (paternal AA and maternal AB call) is shown in dark grey triangles. Note the distinctive pattern for the maternal meiotic trisomy of chromosomes 1 and 16. **Panel C:** BAF of category 3 SNPs: paternal informative SNPs. Subcategory 3A (paternal AB and maternal BB call) is shown in light grey circles and subcategory 3B (paternal AB and maternal AA call) is shown in dark grey triangles. **Panel D:** BAF of category 4 SNPs: obligate homozygous SNPs. Subcategory 4A (paternal BB and maternal BB call) is shown in light grey circles and subcategory 4B (paternal AA and maternal AA call) is shown in dark grey triangles. These SNPs are primarily used for quality control: for detection of contamination and inspection of the noise for homozygous SNPs. **Panel E:** Detail of Category 1 SNPs of chromosome 1 shown in panel A. **Panel F:** Detail of category 2 SNPs of chromosome 1 shown in panel B. **Panel G:** Detail of category 3 SNPs of chromosome 1 shown in panel C **Panel H:** Detail of category 4 SNPs of chromosome 1 shown in panel D.

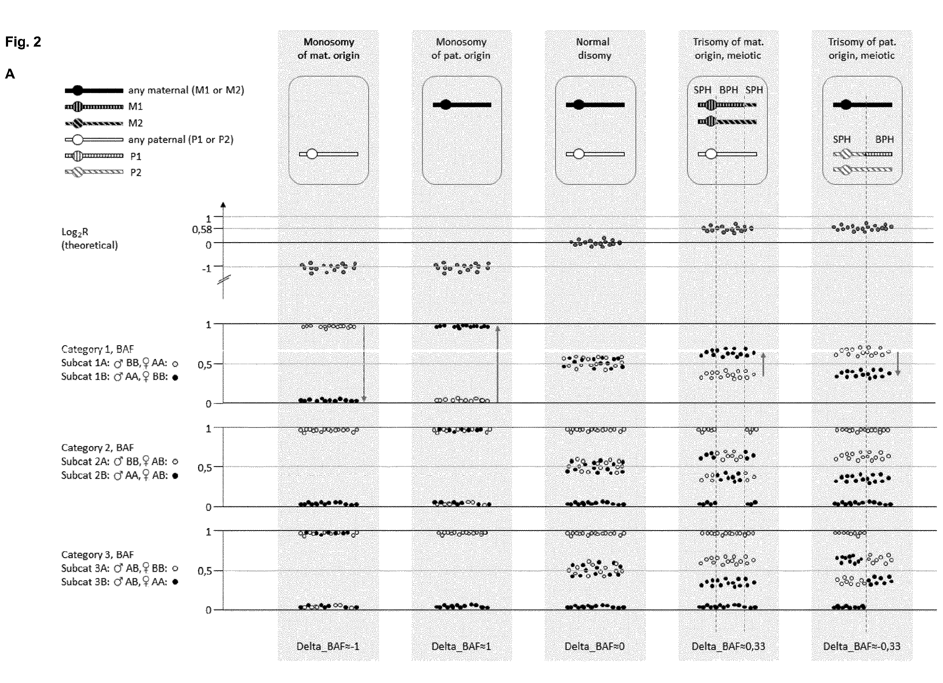

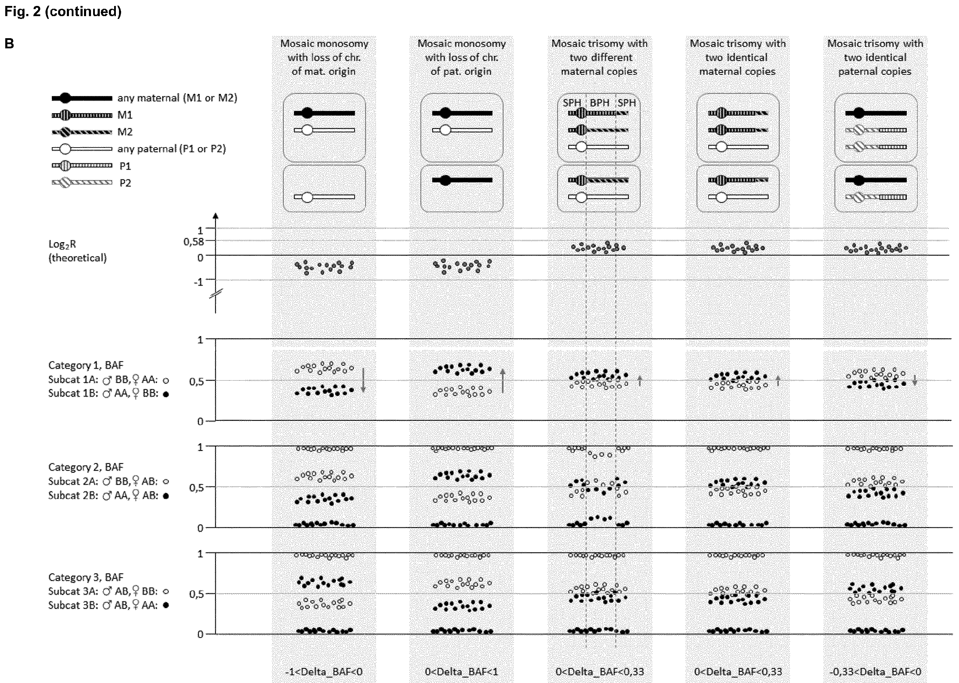

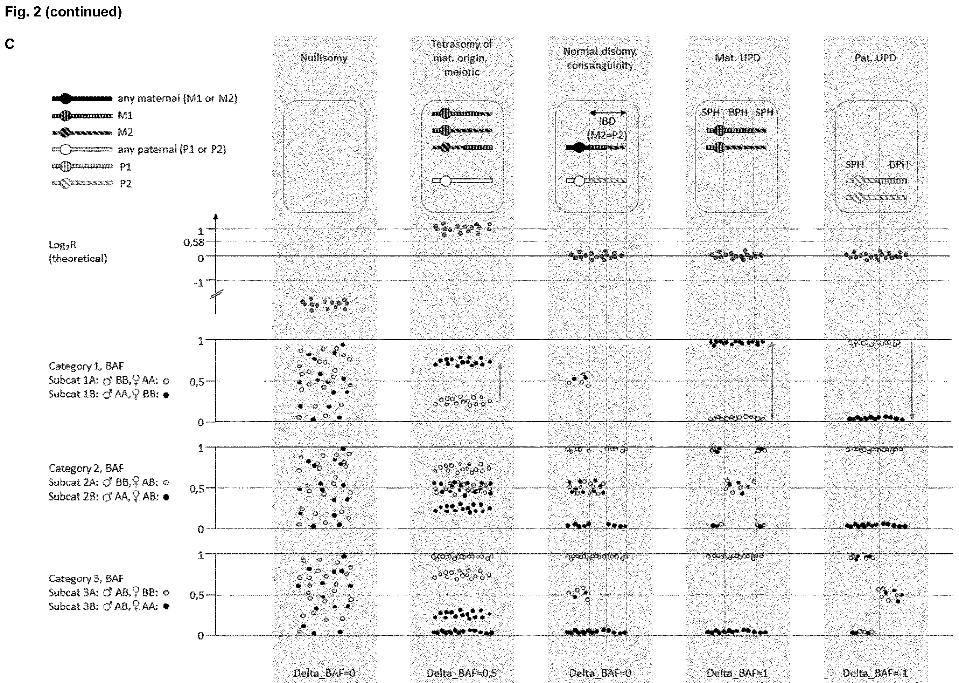

**Figure 2.** Schematic overview of expected $\log_2 R$ and BAF profiles for the most common whole chromosome anomalies. For clarity the number of AF values is greatly reduced. The 'delta_BAF' value is calculated based on the difference in mean BAF of both subcategories of category 1 SNPs. Note that all mentioned chromosomal anomalies can be discriminated using a combination of $\log_2 R$ values and the patterns observed for the BAF of category 1, 2 and 3 SNPs. Even though no haplotyping is used meiotic and mitotic trisomies can be discriminated. 'M1: maternal haplotype 1, 'M2': maternal haplotype 2, 'P1': paternal haplotype 1, 'P2': paternal haplotype 2. 'Male': paternal genotype, 'Female': maternal genotype, 'IBD': region that is identical by descent with female and male partner sharing a haplotype, 'SPH': single parental homolog present, 'BPH': both parental homologs present. Mat.: maternal; pat.: paternal.

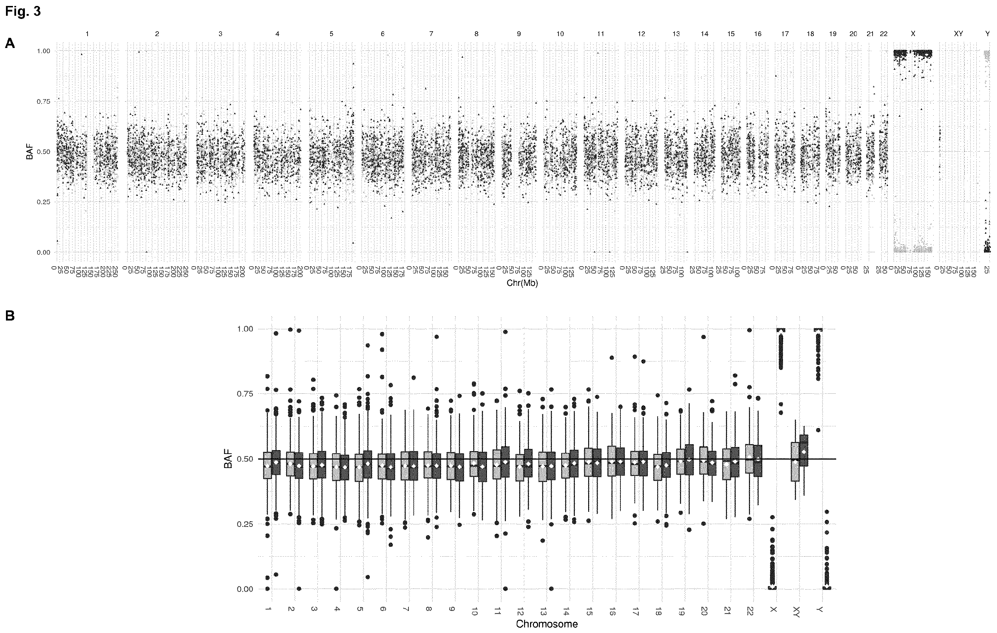

**Figure 3.** Example of a euploid male sample with a higher signal for A compared to B alleles. **Panel A** The BAF for the category 1 SNPs and SNPs on Y are plotted against the genomic position in Mb. The BAF for these SNPs closely relates to the parental contribution. The paternal contribution is reflected by the BAF for the light grey SNPs (subcategory 1A and SNPs on Y) and the maternal contribution is reflected by the BAF for the dark grey SNPs (subcategory 1B and SNPs on Y). **Panel B.** Boxplots for the data visualized in panel A. For each chromosome the boxplot for subcategory 1A SNPs is shown left in light grey while the boxplot for the subcategory 1B SNPs is shown right in dark grey. The parameter 'delta_BAF' for a chromosome equals the difference between the means of both subcategories represented by a white dot ($\text{mean}_{\text{BAF subcategory 1B}}$ - $\text{mean}_{\text{BAF subcategory 1A}}$). The delta_BAF is used to estimate the parental contribution and to estimate the degree of mosaicism (if applicable). Note that the mean (white diamond) and median BAF are <0.5 (black line) for most autosomes for both 1A and 1B subcategories.

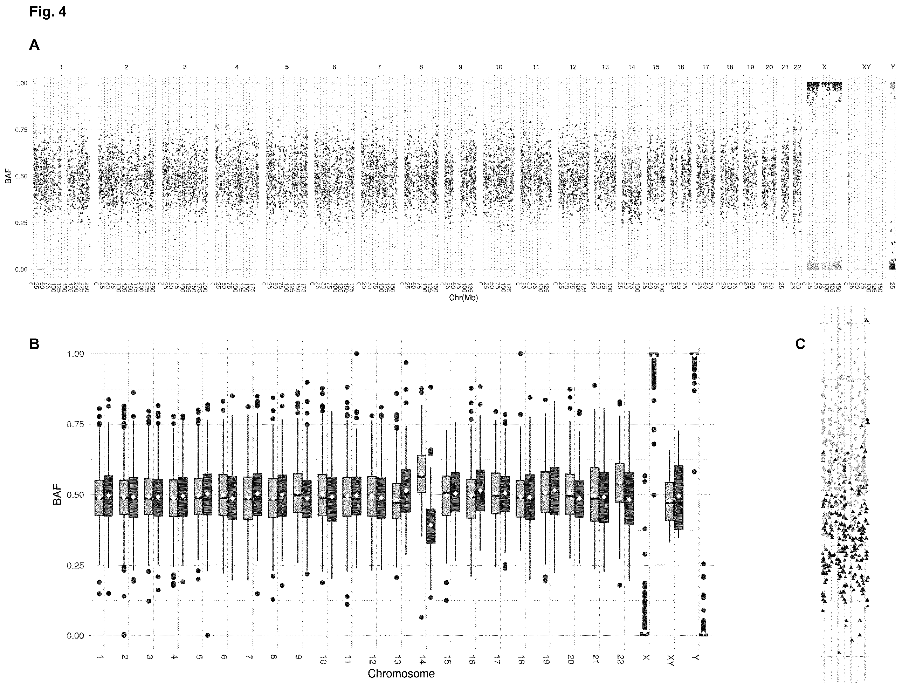

**Figure 4.** Results obtained with APCAD category 1 SNPs (panel A to C) using raw SNP data from a male embryo with a mosaic trisomy 14. The mosaic trisomy could be missed on the raw BAF chart (data not shown). Chromosome 14 stands out when analyzing category 1 SNPS (panels A to C). **Panel A** The BAF for the category 1 SNPs is plotted against the genomic position in Mb. The BAF for these SNPs closely relates to the parental contribution. The paternal contribution is reflected by the BAF for the light grey SNPs (subcategory 1A) and the maternal contribution is reflected by the BAF for the dark grey SNPs (subcategory 1B). As $\log_2 R$ shows an increase in copy-number for chromosome 14 (data not shown) the result is interpreted as a paternal (mosaic?) trisomy. The X is of maternal origin while the Y is of paternal origin as can be expected for a male embryo. **Panel B.** Boxplots for the data visualized in panel A. For each chromosome the boxplot for subcategory 1A SNPs is shown left in light grey while the boxplot for the subcategory 1B SNPs is shown right in dark grey. The parameter 'delta_BAF' for a chromosome equals the difference between the means of both subcategories represented by a white dot ($\text{mean}_{\text{BAF subcategory 1B}}$ - $\text{mean}_{\text{BAF subcategory 1A}}$). The delta_BAF is used to estimate the parental contribution and to estimate the degree of mosaicism (if applicable). **Panel C.** Detail of panel A for chromosome 14.

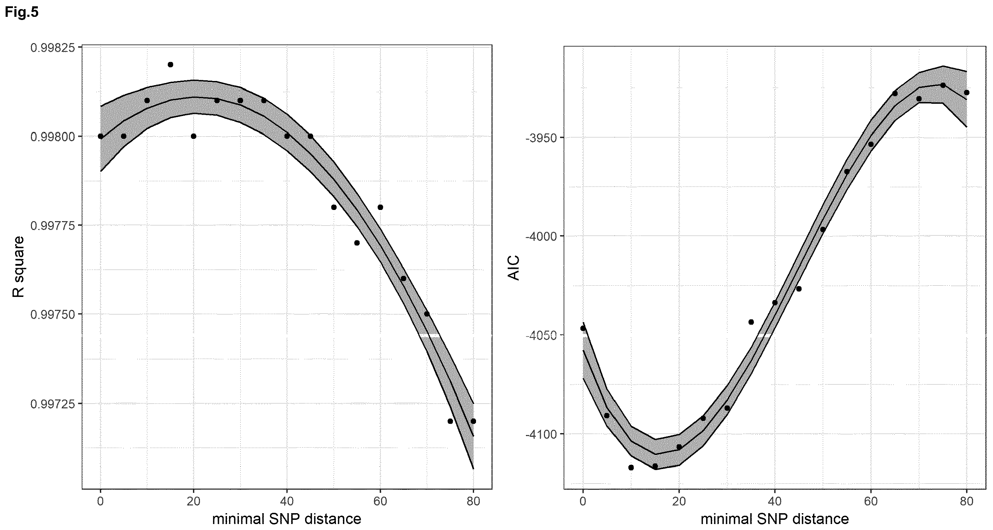

**Figure 5.** $R^2$ scores from second order generalized linear models with different minimal distance between SNPs to remove highly interdependent data shows best fit when the minimal distance between subsequent SNPs (minimal SNP distance) is between 10 and 35 kb (left panel, maximal $R^2$) or between 10 to 20 kb (right panel; minimal AIC score). We used a minimal distance of 20 kb between subsequent SNPs for further analysis.

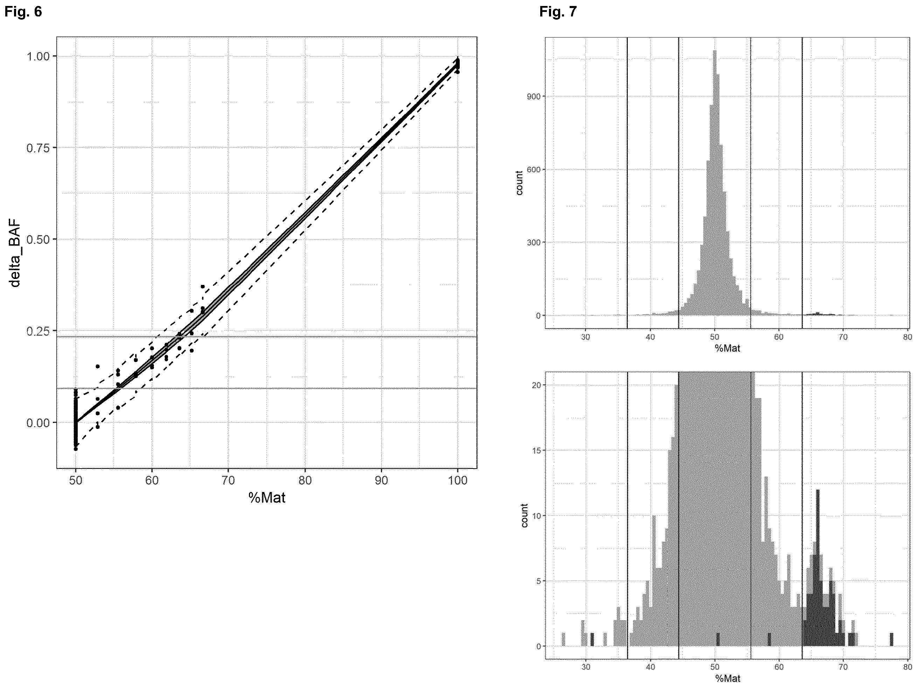

**Figure 6.** Relation between percentage maternal contribution (%Mat) and delta_BAF from the cell mixture experiment after removal of closely clustered SNPs <20kb. Here cells from a 47,XY,+21 cell line were tubed together with cells from a cell line from a sib with a 46,XY karyotype. The %Mat was calculated based on the input while the delta_BAF was calculated from the SNP array data after removal of closely clustered SNPs. The central line corresponds to the best fit which was obtained using a second order generalized linear model. The 95% confidence interval for the model is also shown (outer full lines). The outer dashed lines indicate the prediction interval. Grey horizontal lines indicate cutoffs of delta_BAF at 0.0924 and 0.2341 corresponding with the estimated delta_BAF by the model for 25% and 75% of cells with trisomy respectively (%Mat equal to 55.6% and 63.6% respectively).

**Figure 7.** Stacked histogram with bins of width 0.5% for maternal contribution (%Mat) observed for 59 autosomes with a BPH trisomy (dark grey) and 7436 chromosomes for which no clear chromosome anomaly is detected (light grey). Vertical black bars indicate cut-offs at 36.4, 44.4 55.6 and 63.6 %Mat. Upper panel: the maternal contribution for autosomes without detected anomalies approximates the shape of a normal distribution. Lower panel: same data topped at 20 counts showing that BPH trisomies show a skewed parental contribution of under 36.4 or over 63.6 %Mat in 57 out of 59 BPH trisomies (96.6%).

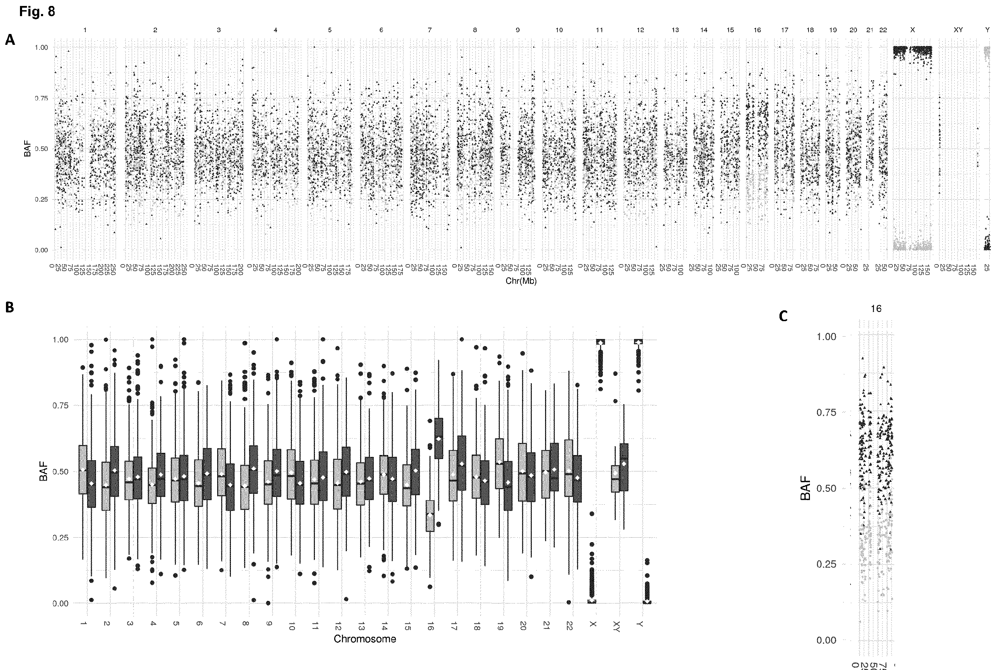

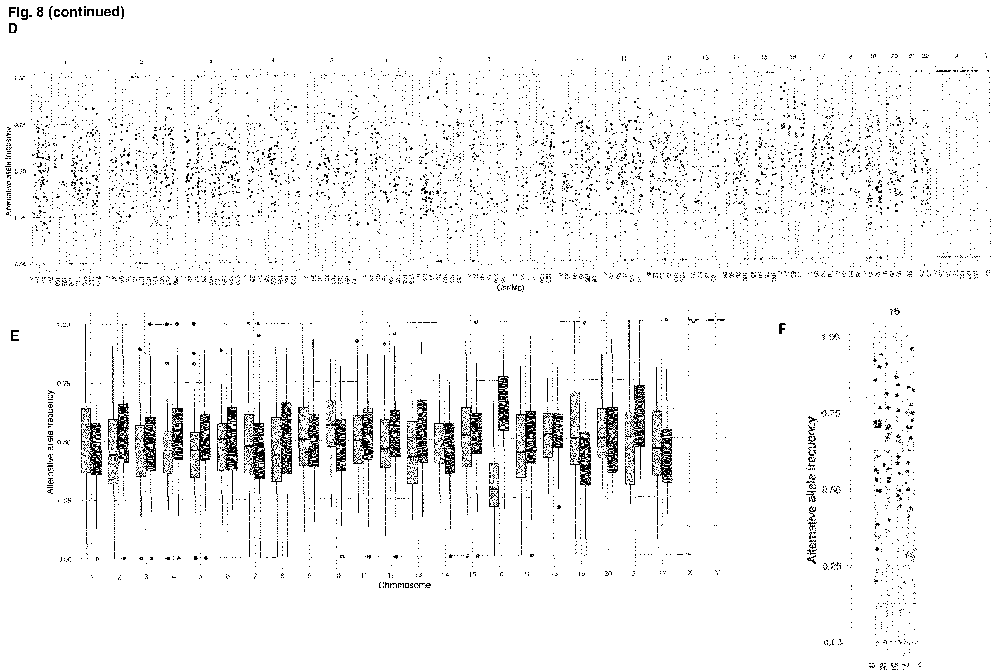

**Figure 8.** SNP array and sequencing results obtained for a male embryo with a meiotic trisomy of chromosome 16 (two maternal haplotypes detected with SNP array). Data for category 1 SNPs and SNPs on Y obtained with SNP array (panels A, B and C) and NGS (panels D,E and F). SNPs of subcategory 1A (father homozygous alternative allele; mother homozygous reference allele or no reads and located on Y) are shown in light grey (left in panel B and E) while SNPs of subcategory 1B (father homozygous reference allele; mother homozygous alternative allele or no reads and located on Y) are shown in dark grey (right in panel B and E). The parameter 'delta_ALAF' equals the difference between the means of both subcategories represented by a white dot in panel E (mean cat. 1B - mean cat. 1A). The delta_ALAF is used to detect aneuploidy and estimate the degree of mosaicism (if applicable).

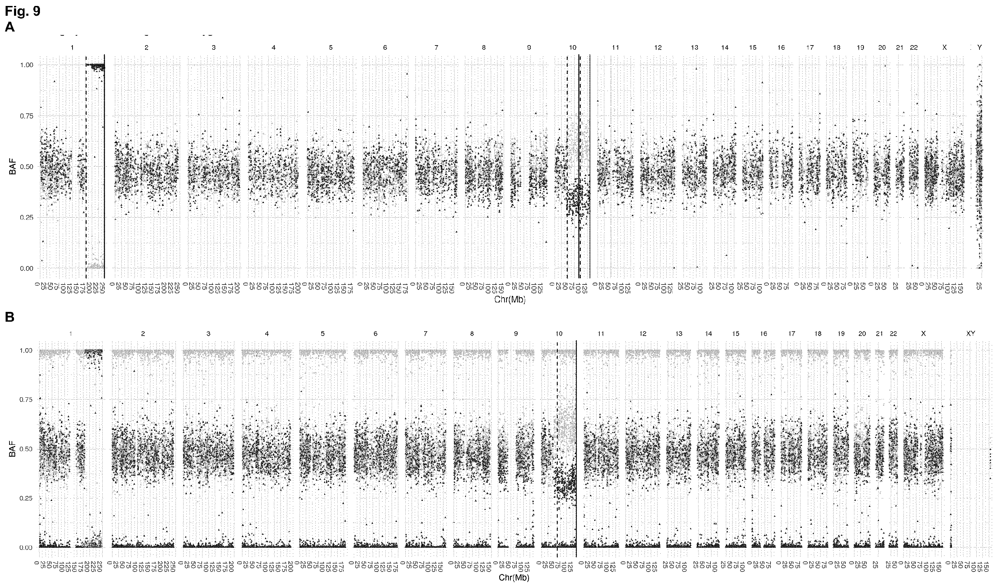

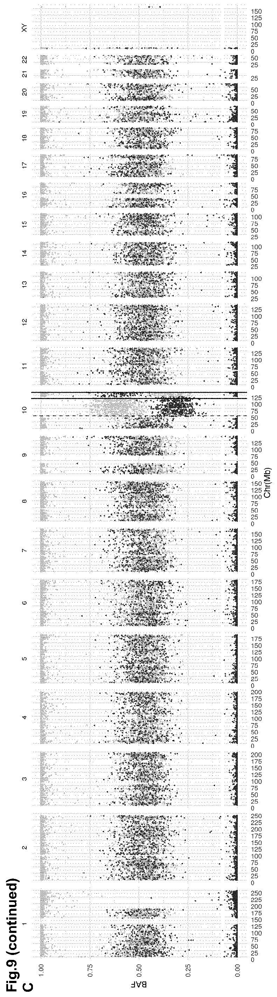

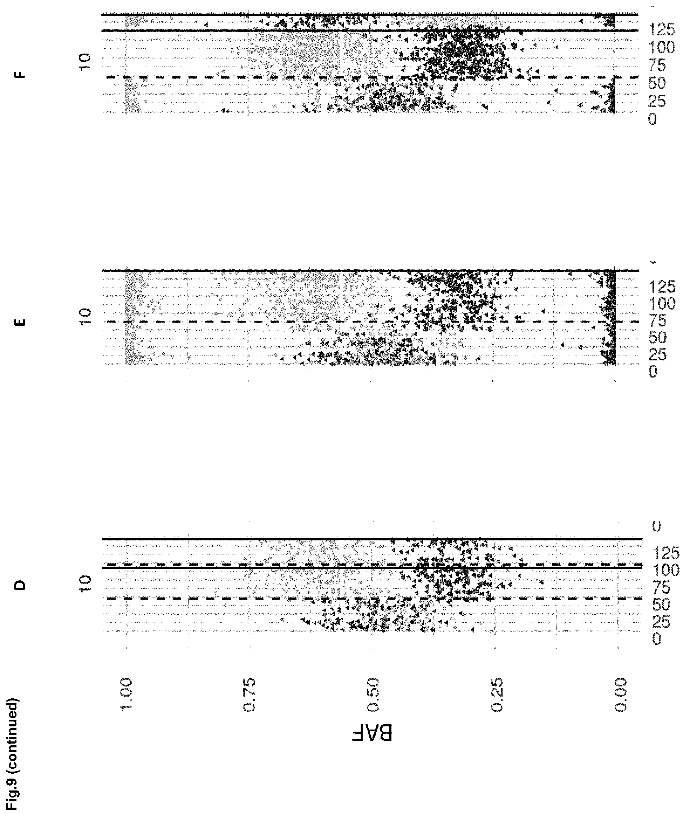

**Figure 9.** Segmentation algorithms applied on category 1 SNPs and SNPs on Y (panels A ,D), category 2 SNPs (panels B, E) and category 3 SNPs (panel C, F) SNPs in a sample with a paternally inherited unbalanced translocation (der(10)t(1;10)(q41;q24)) resulting in a copy number loss on chromosome 1q41 to 1qter and a BPH copy number gain on chromosome 10q24 to 10qter. The left border of a segment with a detected anomaly is shown with a vertical dashed line while the right border of the segment is a full vertical line. Further optimization is required for detection of the segment breakpoints. Panels A, B and C show a genome wide view, panels D, E and F show only the BAF of SNPs for chr 10.

## DETAILED DESCRIPTION OF THE INVENTION

As already outlined above, the present invention is directed to methods for the analysis of genetic material in a subject to evaluate whether a genetic anomaly is present in the genetic material of the subject. Typical for the present invention is that only unphased genotype information of polymorphic variants of a first and a second parent of the subject is needed. Hence, in the present methods the use of phased genotype information, and thus the use of genotype information from a reference sample has become unnecessary. Only genetic samples of both parents of the subject are needed.
Further, the methods of the present invention are preferably applied to samples containing low amounts of target nucleic acids, also referred to as genetic material.
In particular embodiment, the inventors of the present application have identified a new method for aneuploidy detection in genetic material.
The methods of the present invention thus allow using DNA from both parents only, without a phasing reference sample. This is different from other SNP based methods which rely on haplotyping such as siCHILD (e.g. WO2015028576). In the methods already known in the prior art, the use of a DNA sample from a closely related family member is required from each parent to perform reliable aneuploidy detection. This is typically a sibling or a maternal and a paternal grandparent. These samples are a burden to come by as the parents do not always wish to disclose the need for pre-genetic testing and/or in-vitro fertilization to both sides of the family. In other cases, the (prospective) grandparents are deceased or contact has been lost. Also in situations where pre-genetic testing is offered with SNP array or sequencing with the use of one grandparental reference (e.g. maternal side), aneuploidy can now be detected without the need to ask a sample from a grandparent from the other parent's side (e.g. parental side). In addition, it greatly improves aneuploidy detection when performing karyomapping which is neither intended or validated for aneuploidy detection.
As used herein, the term "*allele*" is used herein to refer generally to one copy of a naturally occurring gene or a particular chromosome region in a diploid subject. As diploid subject has two sets of chromosomes and two copies of a particular gene, and thus two haplotypes of any region of the chromosome and two alleles of any polymorphic site within the gene or chromosome region.
The term "*haplotype*" means a combination of genetic (nucleotide) variants in a genomic DNA region on a single chromosome found in an individual or an mRNA derived from a single chromosome found in an individual. Thus, a haplotype includes a number of genetically linked nucleotide variant markers (or polymorphic variants) which are typically inherited together as a unit.

Where in embodiments of the present invention reference is made to a "***polymorphic variant***", reference is made to polymorphic variants which may be bi-allelic or multi-allelic genetic variants segregating in a family or population, without any cutoff on the minor allele frequency in the population.

Where in embodiments of the present invention reference is made to "***allele frequency***" reference is made to the fraction of one allele over the total amount of alleles in the DNA sample following genotyping. In this context allele frequency is similar to polymorphic variant allele frequency. In a preferred embodiment, the allele frequency refers to B allele frequency (BAF) that is the fraction of B alleles in the polymorphic variant-typing data, which may be obtained from a DNA sample by high-throughput genotyping methods, e.g. SNP-arrays or sequencing technologies. In a preferred embodiment, the allele frequency is a B allele frequency. Evidently, when a claim or embodiment of the invention refers to a B allele frequency, A allele frequencies could be used as well. B allele frequencies comprise A allele frequency information and vice versa.

In general, an AF value is expressed using a value from 0 to 1, as they refer to the frequency or fraction. In principle, AF values may be expressed using a multiplicity of said value, e.g. using a value from 0 to 100. For example, an AF value of 0.5 that indicates that half of total amount of alleles has the specific polymorphic variant allele, may be expressed as e.g. 50. In that instance, an AF value of 1 (i.e. all alleles have the particular genotype) will be expressed as 100.

Where in embodiments of the present invention reference is made to ***high throughput genotyping technologies***, reference is made to any massively parallel sequencing, SNP-array or next-generation sequencing technologies. A high throughput genotyping technology provides, after initial processing, raw polymorphic variant data, including genotype calls, DNA quantity values and AF values.

"***DNA quantity values***" refer to high throughput genotyping measurements that indicate the quantity of genetic material present in the sample. Typical DNA quantity values obtained using SNP-array genotyping are logR values. Typical DNA quantity values obtained using sequencing technologies are normalized binned read counts and/or logR values. DNA quantity values may have been determined locally or genome-wide. Preferably, genome-wide DNA quantities values are combined with the method of the present invention. Normalization of DNA quantity values refers to a process that normalizes the raw DNA quantity values (e.g. logR-values). In general, this procedure can consist of: (1) correction for %GC-bias in the raw values, (2) detection of the likely normal disomic chromosomes, and (3) trimmed mean (or median) correction on the basis of the detected normal disomic chromosomes.

Where in embodiments of the present invention reference is made to ***sequencing technologies***, reference is made to any next-generation sequencing methodology, e.g. whole-genome sequencing, exome sequencing, targeted sequencing.

***Genotype information*** in general refers to the genetic makeup of a subject. Genotype information may be phased or unphased genotype information. The method according to the different embodiments of the invention is typically characterized in that unphased genotype information of a first and second parent is used. The genotype information of the first and second parents (also referred to as parental genotype information) may be obtained directly by genotyping a sample obtained from the parent (e.g. by massive parallel sequencing or SNP array-typing of a parental blood or tissue sample. Preferably, the parental genotype information is available as discrete polymorphic values.

The term "***genotype***" as used herein means the nucleotide characters detected in a given sample for a particular polymorphic variant marker.

A ***phased genotype*** or phasing of genotypes of the parent(s) can be attained by the use of a close relative, e.g. grandparents and/or a sibling and/or one or a few embryos. In the phasing process the close relative is used to determine which alleles of polymorphic variants are located on the same chromosome copy (*in cis*) in the reference. The method described here does not use phased genotypes.

An ***unphased genotype*** is obtained by the use of only the genotype information of the parents. When the term unphased genotype is used in connection with a biallelic gene (or a particular chromosome region), unphased genotypes for a particular marker can be expressed, e.g. in the form of X/Y, wherein X is the genotype in one allele while Y is the genotype in the other allele, with allelic specificity undefined. That is, it is undetermined which of the two alleles X is associated with, and Y is associated with. Unphased genotypes for a plurality of markers can be X/Y at a marker, A/B at another marker, C/D at yet another marker, and so one.

In the context of the present invention, a ***parent*** refers to a biological parent of the subject. In the instance of a so-called three-parent embryo/fetus/child, wherein two parents contribute to the chromosomal DNA and a third party contributes the mitochondrial DNA, a parent refers to a parent contributing to the chromosomal DNA.

***Homozygous, hemizygous*** and ***heterozygous*** as used in the present application are used to describe the genotype of a diploid organism at a single locus on the DNA. Homozygous describes a genotype consisting of two identical alleles at a given locus. Heterozygous describes a genotype consisting of two different alleles at a given locus. Hemizygous describes a genotype consisting of only a single copy at a given locus in an otherwise diploid organism.

When reference is made to the **parental contribution,** this is used as a general term to indicate the contribution from parent 1 or parent 2 between two genomic locations. The contribution from parent 1 or 2 indicates the proportion of genetic material in the analyzed sample between two genomic locations that originates from parent 1 or parent 2 respectively.

The **parent 1 and parent 2 copy number** indicates the chromosome copy number originating from parent 1 or parent 2 respectively between two genomic locations.

Genetic anomalies characterized by the presence of 2 or more identical chromosome copies originating from one parent such as uniparental isodisomy, and copy number gains (e.g. trisomy, duplication, insertion) are referred to as **single parental homolog (SPH) anomalies.**

Genetic anomalies characterized by the presence of at least 2 different chromosome copies (with hence two haplotypes for at least one chromosome segment) originating from one parent such as uniparental heterodisomy, and copy number gains (e.g. trisomy, tetrasomy, duplication, insertion and copy number gains resulting from an unbalanced translocation) are referred to as

**both parental homolog (BPH) anomalies.**

Chromosome segments characterized by the presence of at least 2 different chromosome copies (and hence two haplotypes for this chromosome segment) originating from one parent are referred to as **both parental homolog (BPH) chromosome segments.**

Wherein in embodiments of the present invention reference is made to a sample comprising genetic material (in particular a DNA sample), reference is generally made to all samples comprising genetic material derived from: a single cell, a few cells, a large-number of cells or cell-free DNA. A sample comprising genetic material may also refer to a cell-free sample obtained from a body fluid sample. In a preferred embodiment, the sample comprises a low amount of genetic material (in particular DNA) of the subject, such as a sample comprising only one or a few cells of the subject. In another preferred embodiment, the sample is a plasma sample obtained from a mother pregnant with said subject.

As is evident from the description, the methods of the present invention are preferably applied to samples containing low amounts of target nucleic acids, also referred to as genetic material. In particular, said genetic material of interest is either present within one or a few target cells, or as free circulating material in the sample. Thus in a particular embodiment, said sample contains one or a few target cells. In a further embodiment, said sample contains one target cell. In another embodiment, said sample contains a few target cells, in particular 1 to 30, more in particular 3 to 20, even more preferably 4 to 12, target cells, for example, 3 to 20, 3 to 15, 3 to 12, 3 to 10, 3 to 8, 4 to 20, 4 to 15, 4 to 12 one, two, three or four target cells. In another particular embodiment, target nucleic acids are present in an amount of 2 ng or less in said sample, in particular 1 ng or less, more in particular 0.5 ng or less. In another embodiment, target nucleic acids are present in an amount of 250 pg or less in said sample; in particular 200 pg or less; more in particular 150pg or less. In another particular embodiment, said target nucleic acids are present in an amount of 100 pg or less; in particular in an amount of 50 pg or less; more in particular in an amount of 30 pg or less. In another particular embodiment, said target nucleic acids are cell-free, circulating nucleic acids. For example, circulating cell-free fetal DNA from a maternal sample, or circulating tumor DNA from a patient sample. While genetic material (e.g. maternal DNA) may be abundant in such samples, target DNA (i.e. genetic material of the subject to be researched, e.g. fetal DNA) is present in only very limited amount. In a particular embodiment, target nucleic acids are present as cell-free nucleic acids in a fluid sample. In particular, said cell-free nucleic acids are present in a fluid sample comprising additional (non-target) nucleic acids. In a particular embodiment, said sample comprises a mixture of target and non-target nucleic acids. Preferably, said target nucleic acids are present in an amount between 0.1 and 20% of said non-target nucleic acids. In another particular embodiment, said sample comprises a mixture of target and non-target nucleic acids, wherein said target nucleic acids are present in an amount of 700 ng or less, in particular 500 ng or less, more in particular 300 ng or less, even more in particular 200 ng or less, 100 ng or less, or 50 ng or less. In yet another embodiment, said sample comprises cell-free nucleic acids, wherein said cell-free nucleic acids are present in an amount as defined herein above.

In a particular embodiment, providing a sample comprising a low amount of target nucleic acids comprises isolating one or a few target cells. The method of the invention may thus further comprise lysing on or a few target cells.

A *single-cell (DNA) sample* refers to a (DNA) sample that is derived from a solitary cell of any tissue or cell type. Since a single cell only contains a few picogram of DNA, methods involving single-cell samples preferably comprise (whole genome) amplification for genotyping of the polymorphic variants. Few-cell (DNA) sample refers to a (DNA) sample that is derived from a few cells of any tissue or cell type. Depending on the number of cells used for DNA extraction, methods involving such a sample may comprise (whole genome) amplification for genotyping of the polymorphic variants. Multi-cell (DNA) sample refers to a DNA sample that is derived from a large number of cells of any tissue or cell type.

*Cell-free (DNA) sample* refers to a sample that is derived from a fluid specimen that contains circulating genetic material. This can refer to cell-free fetal DNA that freely circulates in the maternal blood stream (also called free fetal DNA, ffDNA). An example of such a sample is a body fluid sample (in particular a blood, plasma or serum sample; more in particular a plasma sample) obtained from a pregnant female and comprising a mixture of maternal and fetal genetic material. Another embodiment of a cell-free sample refers to cell-free tumor DNA that freely circulates in the patient's blood stream.

The sample is preferably obtained from a eukaryotic organism, more in particular of a mammal. In a further preferred embodiment, said sample is from non-human animal (hereinafter also referred to as animal) origin or human origin. In a particular embodiment, said animal is a domesticated animal or an animal used in agriculture, such as a horse or a cow. In a further particular embodiment said animal is a horse. In another particular embodiment, said sample is of human origin; In yet another particular embodiment, said sample is obtained from a pregnant woman. In another embodiment, said sample is obtained from a patient suspected from having a tumour or cancer. In another particular embodiment, said cell is a eukaryotic cell, in particular a mammalian cell. In a more particular embodiment, the origin of said cell is as described according to preferred embodiments regarding the sample origin as described above. In another particular embodiment, said target nucleic acids are of eukaryotic origin, in particular of mammalian origin. In a more particular embodiment, said target nucleic acids are as described according to the preferred embodiment regarding the sample origin. Relating thereto, in a preferred embodiment, said target nucleic acids originate from an embryo or a foetus. In another preferred embodiment, said target nucleic acids originate from a (suspected) cancer or tumour cell.

The methods of the present invention are applicable on any cell type. Preferred cells are polar bodies, blastomeres, trophectoderm cells from blastocysts, chorionic villus samples or amniocytes. Preferred genetic material comprises DNA; preferably genomic DNA. In another embodiment, the preferred genetic material comprises cell-free DNA. Preferably the cell-free foetal DNA is from maternal blood, plasma or serum. Both intact foetal cells and foetal cell-free nucleic acids (e.g. DNA) can be identified in maternal blood. The primary source of most foetal cell-free nucleic acids in the maternal circulation is thought to be apoptosis of placental cells. As already mentioned herein above, the methods are applied on a small number of these cell types, i.e. on a few cells, in particular up to 50 cells; more in particular selected from 1, 2, 3, 4, 5, 6, 7, 8, 9, 10 or more cells up to 30; even more in particular on one or two cells. When applied on trophectoderm, said few cells may be selected from 1, 2, 3, 4, 5, 6, 7, 8, 9, 10, or more cells; in particular up to 50 trophectoderm cells.

For the removal of the appropriate of at least one cell, the zona pellucida at the cleavage and blastocyst stages can be breeched by mechanical zona drilling, acidified Tyrodes solution or laser. In preferred embodiments, few cells are preferably multiple trophectoderm cells, preferably human or animal trophectoderm cells. In particular embodiments, the genetic testing is applied for diagnostic testing, carrier testing, prenatal testing, preimplantation testing, or predictive or presymptomatic testing. In these particular embodiments, genetic testing assists to help patients achieve success with assisted reproduction. In another particular embodiment, the methods of the invention are applied for newborn screening. In yet another particular embodiment, the methods of the invention are applied for forensic testing.

"*Genome-wide*" as used herein means that the methods are applied to and provide information on sequences throughout the genome. In particular, the methods of the present invention provide information regarding all chromosomes for which at least fragments are present in the sample. In a particular embodiment, "genome-wide" refers to information regarding all chromosomes for which at least fragments are present in the sample. In a particular embodiment, "genome-wide" refers to information regarding at least one variant per 100 Mb, in particular at least one variant per 10 Mb, in particular at least one variant per 1 Mb throughout the genome. In a further embodiment, it is meant at least one variant per window of 100 Mb, in particular at least 1 variant per window 50 Mb, more in particular at least one variant per window of 10 Mb throughout the genome. In another particular embodiment, genome-wide refers to information regarding at least one variant per window of 10 Mb throughout the genome. In another particular embodiment, genome-wide refers to information regarding at least one variant per window of 1 Mb. In a further embodiment, genome-wide refers to information regarding at least one variant per window of 100 kb, in particular at least one variant per window of 50 kb, more in particular at least one variant per window of 10 kb throughout the genome. In yet another embodiment, genome-wide refers to information regarding at least one variant per window of 1 kb.

"*Genetic anomaly*" refers to any abnormality in the genome. Genetic anomaly detection may involve detecting the presence or absence of genetically normal or abnormal chromosomes or chromosome regions. Genetic anomalies may be detected directly or indirectly. Direct detection of anomalies e.g. comprises direct detection of missing, extra, or irregular portions of chromosomal DNA. The method described here aims to detect genetical anomalies with an unequal maternal and paternal contribution or presence of more than one maternal or more than one paternal haplotype. Indirect detection is not the subject of this invention. As detailed herein, indicating a genetic anomaly may also comprise indicating the meiotic or mitotic origin of said anomaly. In another embodiment, indicating a genetic anomaly refers to providing a map showing the copy number of a chromosome or chromosome region. In a further embodiment, said map covers a whole chromosome. In an even further embodiment, said map covers all autosomes, in particular all chromosomes.

As used in this application, the *trimmed mean AF values* is synonymous for truncated mean AF values and said trimmed mean values involve the calculation of the mean AF values after discarding given parts of a probability distribution or sample at the high and low end, and typically involves discarding an equal amount of both. The number of points to be discarded is usually given as a percentage of the total number of points, but may also be given as a fixed number of points. In other words, before calculating the trimmed mean AF values a small designated percentage of the largest and smallest AF values are removed before calculating the mean. After removing the specified outlier observations, the trimmed mean is found using a standard arithmetic averaging formula. The use of a trimmed mean helps to eliminate the influence of outliers or data points on the tails that may unfairly affect the traditional mean value.

The present invention thus relates to methods for the analysis of genetic material in a subject. In a specific embodiment, the present invention discloses a method for the analysis of genetic material in a subject, said method comprising:

- obtaining unphased genotype information of polymorphic variants of a first and second parent of the subject;
- obtaining the genomic location of the polymorphic variants;
- selection of the polymorphic variants for which the first and second parent are homozygous or hemizygous for a different allele (also referred herein as category 1 polymorphic variants);
- obtaining the allele frequency (AF) values for the selected category 1 polymorphic variants in the genetic material of the subject;
- selection of one allele per polymorphic variant and subcategorization of its corresponding AF frequency of the subject in one of the following subcategories:

  ∘ AF values representing the AF values for alleles present in homozygous or hemizygous state in the first parent (subcategory 1A polymorphic variants);
  ∘ AF values representing the AF values for alleles present in homozygous or hemizygous state in the second parent (subcategory 1B polymorphic variants);

- evaluation whether a genetic anomaly is present in the genetic material of the subject based on the AF values of the subcategory 1A or 1B polymorphic variants and the genomic location of said polymorphic variants.

Said method further comprise calculation of the mean AF values, trimmed mean AF values or the median AF values of the polymorphic variants for a given subcategory 1A or 1B, wherein the polymorphic variants are located between two particular genomic locations on the chromosome, and evaluating whether a genetic anomaly is present in the genetic material of the subject based on said mean, trimmed mean or median AF values of polymorphic variants observed between said genomic locations directly, or optionally by calculating the difference between the median AF values, the mean AF values or the trimmed mean AF values of subcategories 1A and 1B and said difference being indicated as "delta AF", and followed by evaluation whether a genetic anomaly is present in the genetic material of the subject based on the "delta AF" values observed between said genomic locations.

For example, the mean, trimmed mean or median AF values of polymorphic variants is close to 0.5 in euploid samples. In the other hand, when the mean, trimmed mean or median AF value deviates from 0.5 a genetic anomaly is likely to be present, in particular deviation of the mean, trimmed mean or median AF value from 0.5 with a value greater than 0.0462 will be considered as indicative for the presence of a (mosaic) genetic anomaly. For trisomy typically mean, trimmed mean or median AF values above 0.117 or below -0.117 are observed.

For example, the delta AF value is close to 0 in euploid samples. In the other hand, when the delta AF value deviates from 0 a genetic anomaly is likely to be present, in particular a delta AF value above about 0.09 (in particular above 0.0924) or below about 0.09 (in particular below - 0.0924) will be considered as indicative for the presence of a (mosaic) genetic anomaly. For trisomy typically delta AF values above about 0.2 (in particular above 0.2341) or below about - 0.2 (in particular below -0.234) are observed. In still a further embodiment, the AF values, mean AF values, trimmed mean AF values or delta AF values are used to calculate a value for the parental contribution between said genomic locations and evaluating whether a genetic anomaly is present in the genetic material of the subject based on said value for parental contribution observed between said genomic locations. In still a further embodiment, the AF values, mean AF values, trimmed mean AF values, delta AF values or value for parental contribution between said genomic locations and the detected copy number between said genomic locations is used to calculate the copy number originating from parent 1 and the copy number originating from parent 2 between said genomic locations and evaluating whether a genetic anomaly is present in the genetic material of the subject based on said copy numbers originating from parent 1 and parent 2 observed between said genomic locations.

In another aspect, the present invention discloses a method for the analysis of genetic material in a subject, said method comprising:

- obtaining unphased genotype information of polymorphic variants of a first and second parent of the subject;
- obtaining the genomic location of the polymorphic variants;
- selection of the polymorphic variants for which the first parent is homozygous or hemizygous for a specific allele and the second parent is heterozygous for said specific allele (also referred herein as category 2 polymorphic variants) or selection of the polymorphic variants for which the first parent is heterozygous for a specific allele and the second parent is homozygous or hemizygous for said specific allele (also referred herein as category 3 polymorphic variants);
- obtaining the allele frequency (AF) values for the selected category 2 or 3 polymorphic variants;
- selection of one allele per polymorphic variant and subcategorization of its corresponding AF frequency of the subject in one of the following subcategories:

  ∘ AF values representing the AF values for alleles present in homozygous or hemizygous state in the first parent (subcategory 2A polymorphic variants);
  ∘ AF values representing the AF values for alleles heterozygous in the second parent and absent in the first parent (subcategory 2B polymorphic variants);
  ∘ AF values representing the AF values for alleles present in homozygous or hemizygous state in the second parent (subcategory 3A polymorphic variants);
  ∘ AF values representing the AF values for alleles heterozygous in the first parent and absent in the second parent (subcategory 3B polymorphic variants);

- evaluation whether a genetic anomaly is present in the genetic material of the subject based on the AF values of the subcategory 2A, 2B, 3A, or 3B polymorphic variants and the genomic location of said polymorphic variants.

In evaluating whether a genetic anomaly is present, the mean AF values, the trimmed mean AF values or the median AF values of the polymorphic variants for a given subcategory 2A, 2B, 3A or 3B can be calculated, wherein the polymorphic variants are located between two particular genomic locations on a chromosome are used. In euploid samples, said "mean, trimmed mean or median AF" value will be close to 0.5. On the other hand, in case of a genetic anomaly, the "mean, trimmed mean or median AF" value will be different from 0.5, in particular a deviation of mean, trimmed mean or median AF value from 0.5 with a value greater than 0.025 ; in particular when the AF value deviates from 0.5 with a value greater than 0.045; more in particular when the AF value deviates from 0.5 with a value greater than 0.0462 will be considered as indicative for the presence of a (mosaic) genetic anomaly. For trisomy typically AF values deviate from 0.5 with a value above 0.1; in particular above 0.117.

Subsequently, also the difference between the median AF values, the mean AF values or the trimmed mean AF values of subcategories 2A, 2B, 3A or 3B can be calculated and being indicated as "delta AF". This "delta AF" value is then used to evaluate whether an genetic anomaly is present in the genetic material of the subject. In euploid samples, said "delta AF" value will be close to 0. On the other hand, in case of a genetic anomaly, the "delta AF" value will be different from 0, in particular a delta AF value above 0.0924 or below -0.0924 will be considered as indicative for the presence of a (mosaic) genetic anomaly. For trisomy typically delta AF values above 0.2341 or below -0.2341 are observed.

In case the polymorphic variants of category 2 or 3 are used for further genetic analysis in one of the methods according to the present invention, an additional filtering can be applied to the selection of the AF values for the the selected category 2 or 3 polymorphic variants. In said filtering method, the selected AF values of the polymorphic variants of the subcategories 2A; 2B, 3A and/or 3B are further selected using an upper cut-off value and a lower cut-off value. Said cut-off values can be fixed or variable. In one embodiment, the polymorphic variants of category 2 or 3 are further selected using a fixed upper and lower cut-off value. In particular, polymorphic AF values of category 2A and 3A are selected below a specific cut-off value, whereas polymorphic AF values of category 2B and 3B are selected above a specific cut-off value. As a result, the homozygous polymorphic variants will be filtered out.

One way to perform said filtering is to perform a Receiver Operating Characteristic (ROC) analysis, wherein either a general cut-off is used or wherein a cut-off is determined per sample. In case the filter method is applied to polymorphic variants of subcategories 2A and 3A, the following steps can be applied:

- take the AF values of category 1 (representing the true positive and false negative AF values), excluding the AF values from polymorphic variants on X and Y, optionally also excluding the polymorphic variants located within known copy number variations present in the parents;
- for polymorphic variants for which both parents are hemizygous or homozygous for the same allele, the AF value of said allele is taken (representing the true negative and false positive AF values);
- determine the cut-off value using a parameter derived from a ROC analysis (e.g. determine optimal Youden's J statistic) for determining the optimal AF value threshold for determining a heterozygous SNP in which:

  ∘ the true positives are the AF values of the polymorphic variants of category 1 below a threshold for AF;
  ∘ the false negatives are the AF values for the polymorphic variants of category 1 above a threshold for AF.
  ∘ the true negatives are the AF values of variants for which both parents are hemizygous or homozygous for the same allele that are above a threshold for the allele frequency;
  ∘ the false positives are the AF values of variants for which both parents are hemizygous or homozygous for the same allele that are below a threshold for AF;

Optionally, the cut-off value can be different depending on the type of allele determined (e.g. in a SNP array, a different cut-off value can be present for A or B alleles). In this case, the AF values used for the ROC analysis are limited to the AF values of this allele type only.

Optionally, preferably when determining the cut-off value using data from the sample itself, the cut-off value is determined using data which are first categorized per chromosome, obtaining an optimal cut-off value for each chromosome separately and taking the mean, trimmed mean or median of these values as cut-off value for all AF values of category 2 or 3 polymorphic variants. In another embodiment, the polymorphic variants of category 2 or 3 are further selected using a variable upper and lower cut-off value. Even more in particular, the selection of AF values using an upper and lower cutoff value is essential for subcategory 2A, 2B, 3A and/or 3B polymorphic variants.

In case the filter method is applied to polymorphic variants of subcategories 2B and 3B, the following steps can be applied:

- take the AF values of category 1 (representing the true positive and false negative AF values), excluding the AF values from polymorphic variants on X and Y)-, optionally also excluding the polymorphic variants located within copy number variations present in the parents;
- take the AF value obtained in the subject of polymorphic variants or invariable loci for an allele that is not present in any of the parents (representing the true negative and false postive AF values);
- determine the cut-off value using a parameter derived from a ROC analysis (e.g. determine optimal Youden's J statistic) for determining the optimal AF value threshold for determining a heterozygous SNP in which:

  ◦ the false negatives are the AF values of the polymorphic variants of category 1 below a threshold for the allele frequency;
  ◦ the true positives are the AF values of the polymorphic variants of category 1 above a threshold for the allele frequency;
  ◦ the false positives are the AF values of alleles not present in any of the parents that are above a threshold for the allele frequency;
  ◦ the true negatives are the AF values of alleles not present in any of the parents that are below a threshold for the allele frequency.

Optionally, the cut-off value can be different depending on the type of allele determined (e.g. in a SNP array, a different cut-off value can be present for A or B alleles).

Optionally, when determining the cut-off value using data from the sample itself, the cut-off value is determined using data which are first categorized per chromosome, obtaining an optimal cut-off value for each chromosome separately and taking the mean, trimmed mean or median of these values as cut-off value for all AF values of category 2 or 3 polymorphic variants.

In another embodiment, the polymorphic variants of category 2 or 3 are further selected using a variable upper and lower cut-off value. In said instance, a given percentile for the AF values obtained in the subject for alleles present in homozygous state in both parents of the subject is calculated. Then, the AF values of polymorphic variants in the subject with an AF value lower than the determined cut-off value for alleles present in homozygous state in one parent and in heterozygous state in the other parent are selected (categories 2A and 3A polymorphic variants). Further, a given percentile for the AF values obtained in the subject for alleles not present in both parents of the subject is calculated. Then, the AF values of polymorphic variants in the subject with an AF value higher than the determined cut-off value for alleles not present in one parent and in heterozygous state in the other parent are selected (categories 2B and 3B polymorphic variants).

For example, a cut-off value is calculated using a predefined value for the false positive rate for detecting a heterozygous genotype. A general cut-off value (same for each sample) can be taken, or a different cut-off value can be determined for each sample. Preferably, a different cut-off value is determined for each sample. The cut-off can further be dependent on the type of the allele that is determined (e.g. in a SNP array, a different cut-off value can be present for A or B alleles). For example, for polymorphic variants for which both parents are hemizygous or homozygous for the same allele, the AF frequency of said allele can be taken. If a 0.1% false positive rate is accepted, the 0.1th percentile of the AF values is taken as a cut-off value. For each type of allele (e.g. A or B allele in the SNP array) a different cut-off value can be determined, depending on the type of allele.

In a different example, polymorphic variants or invariable loci for which both parents are hemizygous or homozygous for the same allele, the AF of an allele different from said allele has to be taken. If a 0.1% false positive rate is accepted, the 99.9th percentile of the AF values is taken as a cut-off value. For each type of allele (e.g. A or B allele in the SNP array) a different cut-off value can be determined, depending on the type of allele.

In some embodiments of the present invention, a number of polymorphic variants are removed from the further analysis in the disclosed methods because said polymorphic variants are clustered. For example, polymorphic variants or SNPs that are distributed less than 50 kb, preferably less than 20 kb from each other are removed from further analysis.

In another embodiment, polymorphic variants are removed from further analysis because they do not meet all quality requirements. For example, polymorphic variants that do not show sufficient intensity will be removed from further analysis.

Apart from the detection of aneuploidy in embryonic samples, the methods of the present invention can also be applied for detection of large mosaic copy number alterations present in genomic DNA. In another aspect, the methods can also be used with whole genomic sequencing data.

In one embodiment of the invention, a report displaying the AF values, the mean AF values, the trimmed mean AF values, the median AF values or the delta AF values obtainable by any of methods of the present invention is disclosed.

In another embodiment, a computer-assisted method for the analysis of genetic material of a subject is provided, said method comprising:

- loading in a computer system unphased genotype information of polymorphic variants of a first and second parent of the subject,
- loading in the computer system the genomic location of the polymorphic variants,
- selecting of the polymorphic variants based on one of the criteria as disclosed for each of the methods of the invention,
- providing by the computer system the AF values, the mean AF value, the median AF values or the delta AF values to indicate a genetic anomaly in the genetic material of the subject.

EXAMPLES

The present invention is now further disclosed using the following examples.

**Example 1: Analysis of parental contribution for aneuploidy detection (APCAD) in preimplantation embryos.**

*Patients, and materials and methods*

*Stimulation, oocyte retrieval, ICSI, embryo culture, biopsy and cryopreservation.*

Described in De Rycke et al. (De Rycke et al. 2020).

*Peripheral lymphocytes, genomic DNA and embryos*

The local ethical committee approved obtaining DNA and cell lines for the cell mixture experiment under number B.U.N. 143201630618. After informed consent from the parents had been obtained, blood was drawn from two siblings and their parents. One sibling was affected with Down syndrome (47,XY,+21; trisomy 21) and the other one was euploid (46,XY). Blood was collected in Sodium Heparin tubes for cell culture and in EDTA tubes for DNA extraction. Fresh peripheral lymphocytes were isolated from whole blood and washed in PBS. For the cell mixture experiment we mixed lymphocytes from the two siblings in different proportions, with a total of eight cells per tube. Four replicate series containing the nine possible combinations were generated (0+8, 1+7, 2+6, 3+5, 4+4, 5+3, 6+2, 7+1, 8+0), see Table 1.

For retrospective analysis we selected embryos with biopsy between September 1 2015 and December 31 2017 that were diagnosed as being not genetically transferable with Karyomapping, for which written informed consent was available and for which the SNP data had passed the QC cut-offs (call rate >85%, <=1% miscall rate). Embryos that were tested for a monogenic disorder (PGT-M, majority) and/or a structural rearrangement (PGT-SR, minority) were included. The data from these embryos were reanalyzed with APCAD. As the SNP data were available after preimplantantation genetic testing with Karyomapping, no additional intervention on the patient or embryo for this study was required. The local ethical committee also approved this study under number B.U.N. 143201731745.

*WGA and SNP array analysis*

We used the Karyomapping workflow for whole genome amplification (WGA) and SNP array analysis according to the manufacturer's recommendations (Vitrolife).

*Statistical analysis and curve fitting*

We used R and RStudio for statistical analysis. Generalized linear models were generated using the R Stats package.

**Results**

*Improved visualization using APCAD profiles*

The first part of APCAD involved a more profound analysis of the raw BAF (rBAF) using the available SNP data. Therefore we defined different SNP categories based on the parental genotypes (Table 2) and visualized the BAF for each category separately (cBAF). Figure 1 shows results obtained with APCAD for a male embryo with a 47,XY,+1,+16,-22 karyotype. The first category, category 1, consists of SNPs which are expected to be heterozygous in the euploid female embryo: SNPs for which both parents show a homozygous SNP call for a different allele. These SNPs are further classified in a subcategory 1A and subcategory 1B. Subcategory 1A contains SNPs with a paternal BB call and maternal AA call while subcategory 1B contains SNPs with a paternal AA call and maternal BB call (Figure 1A and 1E and Table 2). We also add SNPs on the Y chromosome to this panel for which a BB (category 'other', added to subcategory 1A) or an AA genotype call (category 'other', added to subcategory 1B) is detected for the paternal sample and a very low signal intensity ($\log_2 R < -4$) is obtained for the maternal sample. Note that the BAF for the selected SNPs now reflect the paternal (subcategory 1A) or maternal contribution (subcategory 1B). In the example (Figure 1A) a skewed parental contribution can be observed for chromosome 22 (no maternal copy), chromosomes 1 and 16 (maternal trisomy), X and Y (male embryo). A second category, category 2, are the unphased maternal informative SNPs (heterozygous in mother, homozygous in father; Figure 1B and 1F). A third category, category 3, consists of unphased paternal informative SNPs (heterozygous in father, homozygous in mother; Figure 1C and 1G). A fourth category, category 4, consists of SNPs that are expected to have a homozygous genotype in the test sample (Figure 1D, 1H): SNPs for which both parents have a homozygous call for the same allele (Both AA or both BB). The category 4 SNPs are useful for quality control: for detection of contamination and analysis of the BAF distribution of homozygous SNPs. Also for category 2, category 3 and category 4 SNPs subcategories were defined based on the parental genotypes (Table 2).

The cBAF profiles together with the rBAF and $\log_2 R$ profiles are collectively referred to as APCAD profiles. Inspection of the APCAD profiles allows aneuploidy detection. In the example, even in the absence of a phasing reference, it can be concluded that the monosomy 22, trisomy 1 and 16 are of maternal origin. Recombinations in the chromosomes with a maternal trisomy can be recognized in panels B and F of Figure 1 as alterations of regions without homozygous SNPs (both maternal haplotypes present) with regions that do harbor homozygous SNPs (two identical maternal copies). A specific pattern can also be observed for the heterozygous SNPs in this panel. It can be concluded that the observed trisomies in the example originate from meiosis I based on the pattern observed around the centromeres of chromosomes 1 and 16. A schematic overview of the different expected patterns for the most common whole chromosome anomalies is shown in Figure 2. Segmental anomalies present with the same pattern as the whole chromosome counterparts. E.g. for a duplication the observed pattern is similar to the pattern observed for a trisomy. Obviously, this pattern is only observed for the segment that is duplicated.

*Retrospective analysis using APCAD profiles*

The available SNP array data from 359 embryos were retrospectively analyzed. We generated the cBAF profiles and screened these together with rBAF and $\log_2 R$ profiles for the presence of meiotic or non-mosaic chromosome anomalies without knowledge of the indication and without accessing the available haplotype information. Afterwards, we compared the results from interpretation of these APCAD profiles with the findings obtained with karyomapping ($\log_2 R$, rBAF and haplotype profiles) for detection of *de novo* chromosome anomalies (PGT-A). We use the terms single parental homolog (SPH) and both parental homolog (BPH) copy number gain to distinguish copy number gains that can detected using haplotyping (BPH anomalies) and copy number gains that cannot (SPH anomalies), in line with other publications (Kubicek et al. 2019).

We observed an abnormal ploidy in five samples with both methods. In one near-triploid and two triploid samples three BPH tetrasomies, one UPD (heterodisomy), and 63 BPH trisomies were detected in total. In two haploid samples the absence of a paternal haplotype was observed for all chromosomes. We excluded these five samples with abnormal ploidy from further analysis.

Compared to Karyomapping, we noticed that the APCAD profiles allowed a more sensitive detection BPH anomalies if one haplotype is severely underrepresented. In four samples we observed the presence of a both maternal haplotypes for all chromosomes (n=1) or segments of all chromosomes (n=3). These observations can be explained by contamination with maternal DNA (e.g. from a persisting second or first polar body respectively (Ottolini et al. 2015)) but we cannot rule out a partially rescued triploidy. Please note that in one of these instances a maternal monosomy could be falsely interpreted as a mosaic monosomy. In one sample the presence of a second maternal haplotype for a chromosome segment of approximately 6Mb on chr1 was detected. Based on the APCAD profile, the small size of the BPH segment and its low abundance a contamination with a chromosome segment from maternal origin (e.g. polar body) is suspected but we cannot rule out a partially rescued segmental or whole chromosome BPH copy number gain. We excluded these 5 samples with potential contamination from further analysis.

The remaining 349 embryos without potential contamination and with normal ploidy represent 8376 chromosomes (autosomes, X and Y). All chromosomes (65/65) with *de novo* BPH anomalies that had been identified by the presence of two maternal or two paternal haplotypes using Karyomapping were also identified by analysis of the APCAD profiles. Concordant results were obtained in 348 samples by the detection of 57 autosome BPH trisomies, 5 sex chromosome trisomies, 1 segmental BPH copy number gain and 1 BPH isochromosome. In one sample for one chromosome a segmental BPH copy number gain was observed with both methods but the detected copy number was different: it was interpreted as a gain of one extra copy (+1) with Karyomapping while it was concluded to be a gain of two copies (+2) based on the APCAD profiles.

All chromosomes (90/90) in which a *de novo* non-mosaic copy number loss was identified by the loss of a parental haplotype combined with the absence of heterozygous SNPs on the rBAF profile using Karyomapping were also identified using the APCAD profiles. In 346 samples 62 monosomies, 26 segmental copy number losses and 1 isochromosome were detected concordantly. The sizes of the detected copy number losses ranged between 10 and 123 Mb. In one embryo a non-mosaic copy number loss was identified with both methods but the size of the anomaly was different: a paternal monosomy was detected with Karyomapping while a paternal deletion combined with a mosaic monosomy for the rest of the derivative chromosome was observed based on APCAD profiles. In addition, two anomalies interpreted as a mosaic monosomy with loss of the paternal haplotype in most of the cells with Karyomapping were diagnosed as a paternal deletion in all analyzed cells and a mosaic monosomy for the rest of the derivative chromosome by analysis of APCAD profiles.

The identification of SPH trisomies and segmental SPH copy number gains can be difficult using Karyomapping given the low signal to noise ratio of $Log_2R$ profiles, especially in samples with a lower call rate. Also, the distinction between non-mosaic and mosaic SPH anomalies can be subjective and in contrast to BPH trisomies, meiotic and mitotic SPH trisomies cannot be discriminated. Therefore, Karyomapping cannot be considered the gold standard for detection of non-mosaic SPH anomalies. We compared the APCAD results with Karyomapping only for the sake of completeness with regard to anomalies that are (potentially) of meiotic origin. From 13 SPH trisomies identified with Karyomapping, 12 were identified using the APCAD profiles. One SPH trisomy interpreted by Karyomapping was concluded to be a mosaic SPH trisomy using APCAD profiles. All four SPH segmental copy number gains detected by Karyomapping were also detected using APCAD profiles. In another instance, a chromosome was called as SPH trisomy based on APCAD profiles but not by Karyomapping. A slight increase on $Log_2R$ for that chromosome had been noted by Karyomapping but no (clear) anomalies had been observed on rBAF or haplotype profiles and therefore it was not interpreted as a non-mosaic (SPH) trisomy. Instead it was suspected to be a mosaic SPH trisomy. An overview of discordant and concordant results between Karyomapping an APCAD is shown in Tables 3 and 4 respectively.

For a number of embryos one of the indications for PGT had been a structural rearrangement. Therefore PGT-SR was performed using DNA from a relevant reference with known status, most often a child or parent known to carry the familial structural rearrangement. In this case the structural rearrangement can be identified by the phase of the haplotypes flanking the structural rearrangement breakpoints. This very reliable targeted approach also allows to discriminate balanced from normal segregations. The Karyomapping results for inherited structural rearrangements were compared with the APCAD results that were obtained without knowledge of the indication. We compared the results for segmental anomalies larger than 5Mb (Table 5). Two maternal BPH trisomies, one maternal BPH tetrasomy and a maternal monosomy detected in three embryos using analysis of APCAD profiles were in fact unbalanced translocations resulting in a whole chromosome copy number change. The detected copy number gain on chromosome 15q and copy number loss on 6q in one sample with APCAD corresponded with the Adjacent1 segregation that was observed with Karyomapping for a known 46,XX,t(6;15)(q25.3;q11.1) translocation. An embryo diagnosed with Karyomapping as unbalanced with adjacent2 segregation for the familial 46,XX,t(14;17)(q32.3;q11.2) was interpreted as a deletion on chr17 and a BPH trisomy on chr14 with the APCAD approach. In this sample the presence of a segment (~10Mb) on chr14 with normal copy number was detected with Karyomapping but not with APCAD. The results for the five embryos with inherited unbalanced translocations are summarized in Table 5.

Taken together, we observed a maternal contamination or partially rescued maternal BPH anomaly in 5 embryos by analysis of APCAD profiles that had not been detected using Karyomapping. In five embryos an abnormal ploidy observed. In the remaining 349 embryos all 70 chromosomes including 65 chromosomes with *de novo* BPH anomalies and 5 chromosomes with inherited BPH anomalies detected with Karyomapping were identified by analysis of APCAD profiles. In 68 chromosomes the size (segmental or whole chromosome) and the copy number (+1 or +2 copies) of the detected BPH anomaly was identical with both methods. All 93 chromosomes with a copy number loss larger than 5Mb detected with Karyomapping were identified by analysis of APCAD profiles corresponding with 90 *de novo* and 3 inherited copy number losses. For 92 out of 93 chromosomes the type of non-mosaic copy number loss was identical (segmental or whole chromosome). Out of 17 SPH copy number gains interpreted as non-mosaic by Karyomapping, 16 were interpreted as non-mosaic based on APCAD profiles. In one instance, the SPH copy number detected with Karyomapping was interpreted as a mosaic SPH trisomy using APCAD. Two chromosomes with mosaic anomalies and one chromosome without diagnosis with Karyomapping were interpreted as non-non mosaic anomalies using APCAD. The observed meiotic and non-mosaic anomalies per embryo are summarized for both methods in Table 6.

*Measuring parental contribution*

In a second part of the APCAD method we focused further on the category 1 SNPs (Table 2). As the BAF of an individual SNP is too noisy when analyzing DNA from a few cells (such as a trophectoderm biopsy), we selected SNPs located within a chromosome and we calculated the mean BAF for SNP subcategories 1A and 1B separately. We observed that the mean BAF of both subcategories of SNPs in euploid samples was often just below 0.5 (0.47-0.50) and that the sum was typically lower than 1 (0.95-1.00) (for an example see Figure 3). This indicates that the mean BAF is often underestimated, possibly caused by differences in the detection of A and B fluorophores and the low amount of input material. This phenomenon is equally affecting the BAF of subcategory 1A and 1B SNPs. Hence, we used the difference between the mean BAF obtained for both subcategories for further analysis (mean$_{BAF\ subcategory\ 1B}$ - mean$_{BAF\ subcategory\ 1A}$). This value is from here onwards referred to as the 'delta_BAF'. We expect the 'delta_BAF' to be a more robust parameter compared to the mean BAF of subcategory 1A (or 1B) SNPs. It corresponds with the difference between the means (white diamond) shown in the middle of each box in panel B of Figure 3 and 4. The 'delta_BAF' is close to zero in euploid samples. A positive value is obtained if more maternal than paternal DNA is present while a negative value indicates more paternal than maternal DNA. In the example shown in Figure 4, the delta_BAF is deviating with a value of -0.1882 for chromosome 14. This value, together with an observed increase in Log$_2$R indicates a mosaic paternal trisomy for chromosome 14 in this sample.

We used the delta_BAF to measure the maternal (and hence also the paternal) contribution of a chromosome or chromosome segment. To this end a cell mixture experiment was performed with peripheral lymphocytes from two male siblings of which one was affected with Down syndrome (47,XY,+21) and the other one was euploid (46,XY). Four replicate series containing the nine possible contributions from each sibling were analyzed (see Table 1). After SNP array, the delta_BAF was calculated for all chromosomes. We plotted the results as the measured delta_BAF against the *a priori* known percentage of maternal contribution (%Mat). In theory, for all autosomes except chromosome 21, the %Mat is 50% (no aneuploidy for these chromosomes, n=756) while the %Mat for chromosome X is 100% (n=36) in the male samples, and the %Mat for chromosome 21 is between 50 and 67% depending on the percentage of trisomy 21 cells that were tubed (n=36).

A clear relation between the %Mat and the delta_BAF was observed and curve fitting was performed (data not shown. In order to assign a higher weight to a measurement when the underlying data were less noisy, the weight was calculated as the inverse of the standard error for that delta_BAF value. The standard error of the delta_BAF was calculated as the root of the sum of the squared standard error of the mean of each subcategory (

$$= \sqrt{\sigma_{1B}{}^2/n_{1B} + \sigma_{1A}{}^2/n_{1A}}$$

). Curve fitting showed a better fit for a second order generalized linear model (Akaike information criterion (AIC)=-4046.8) over a first (AIC=-4016.4) order model (p<0.001). A third order (AIC=-4044.9) model did not improve fit over the second order model (p=0.76).

The used whole genome amplification method, MDA (Qiagen Repli-G), generates large fragments of 10 to 100 kb. Therefore we examined if the fit of the curve (Figure 5) improved after removing SNPs which are clustered together and are undergoing the same amplification bias generated by the MDA. The fit improved after removing clustered SNPs with a minimal distance between consecutive SNPs between 10 to 35 kb based on the evaluation of R$^2$ and between 10 to 20 kb based on the evaluation of AIC (Figure 5). We used a minimum 20 kb distance in our subsequent analyses. The fitted curve was used for quantifying the maternal contribution in test samples from the observed delta_BAF (Figure 6).

We assigned cut-offs at the boundaries delta_BAF = 0.0924 and delta_BAF = 0.2341, the delta_BAF predicted by the model for 25% (or 55.6 %Mat) and 75% (or 63.6 %Mat) of trisomic cells respectively. The 0.0924 cut-off is located above the 95% prediction interval for 50.0 %Mat (normal disomy) and the 0.234 cut-off is located at the lower boundary of the prediction interval for 66.6 %Mat (complete trisomy). Below the threshold 0.0924 the value is considered normal (normal disomy). Above 0.234 the value indicates a full trisomy. Between both threshold values the sample is categorized as mosaic. With these cut-offs all chromosomes of the training set with normal disomy (756/756) and complete trisomy (4/4) are correctly categorized. Also chromosomes with trisomy in 50% of the cells (60 %Mat) are expected to be correctly categorized as the prediction interval for 60 %Mat is located between both cut-off values. However chromosomes with lower level mosaic trisomy are potentially categorized as disomy: three out of four samples with one trisomic cell (12.5% trisomy) and one out of four samples with two trisomic cells (25% trisomy) out of eight were categorized as normal disomy. Similarly, chromosomes with high level mosaic trisomy can be labelled as a full trisomy with these cutoffs for the training set: one sample with 75% trisomic cells and three out of four samples with 87.5% trisomy would fall in the (full) trisomy category. All samples with 37.5 and 62.5% trisomy were correctly categorized as mosaic trisomy.

*Retrospective analysis - analysis of parental contribution*

Using the SNP array data from the 349 trophectoderm samples from embryos without contamination and normal ploidy (see section '*Retrospective analysis using APCAD profiles*'), the parental contribution was calculated. We calculated the parental contribution per chromosome for the 22 autosomes, X and Y, resulting in 8376 calculations in total. Between 116 and 1827 SNPs were available for calculation of the parental contribution of a chromosome and each 1A or 1B subgroup contained between 47 and 950 SNPs. We investigated the relation between the calculated parental contribution per chromosome and the whole chromosome anomalies detected using APCAD profiles (*de novo* anomalies, PGT-A ) and Karyomapping (the inherited chromosomal anomalies, PGT-SR).

An overview of the results for the autosomes is shown in Table 7. We detected a very skewed parental contribution larger than 63.6%, which is compatible with over 75% of trisomic cells, in 57 out of 59 (96.6%) of the analyzed autosome BPH trisomies. The median parental contribution was 66.3%, close to the theoretical 66.6%. For 56 BPH trisomies the calculated parental contribution was between 64.2% and 71.28%. There were three outliers with 50.3, 58.5 and 77.7% contribution from the partner that transmitted the trisomy. A very skewed parental contribution was also observed for all (57/57) autosome monosomies with a contribution from one parent of close to 100% (96.7-100%) and for the BPH tetrasomy (1/1) of 74.3% close to the theoretical 75% for a tetrasomy with three maternal copies.

For 7436 autosomes no clear chromosomal anomaly was detected (N.A.D.). For these chromosomes both the mean and median %Mat were 50.2 %Mat. The standard deviation for the %Mat measured for these chromosomes was 2.6 %Mat. For 7136/7436 (96.0%) of these chromosomes the maternal contribution was within the normal range, between 44.4% to 55.6% (Table 7, Figure 7). For 268 chromosomes a moderately skewed parental (maternal or paternal) contribution between 56.7% and 63.6 was observed, a finding compatible with a mosaic and/or segmental chromosomal anomaly that was not detected based on the $\log_2R$ profile. For 32 chromosomes a very skewed parental contribution (>63.6%) was observed. This finding was observed in 9 embryos that contained clear meiotic or mosaic anomalies for other chromosomes and 6 embryos for which no anomalies for other individual chromosomes had been observed. In retrospect, these 6 embryos showed a chaotic pattern on $\text{Log}_2R$ and cBAF plots that was too complicated to assign individual copy numbers. Nevertheless, these embryos are mosaic or aneuploid.

The results concerning the sex chromosomes are summarized in Table 8. For 167 female samples a maternal contribution between 44.4% to 55.6% was observed for 161 X chromosomes while 6/167 (3.7%) X chromosomes showed a maternal or paternal contribution between 55.6 and 63.3% (Table 8). Detection of mosaicism for X or Y chromosomes in male samples using SNPs that are only located on X and Y respectively is not possible. Therefore we also analyzed the maternal contribution for the pseudoautosomal regions (PAR) even though these are relatively small regions (3Mb) and only few SNPs were available: between 9 and 32. Subgroups contained between 3 and 20 SNPs in all but two samples. In these two samples no value for the parental contribution in the PAR was calculated. Given the small number of SNPs, the standard deviation for the measured maternal contribution for the PAR without detected aneuploidy for the sex chromosomes is higher (5.1 %Mat). However a skewed parental contribution over 63.6% was observed for the small PAR region in all XXY trisomies (5/5), X chromosome monosomies (6/6), in a single instances of a terminal deletion on X (1/1) and in one out of three instances with a detected mosaic monosomy X (1/3) while no or limited skewing was observed for 330 out of 334 PAR regions for which no aneuploidy was detected (Table 9).

**Example 2: Use of sequencing technologies instead of SNP array**

*Materials and methods*

Prior to analysis, Multiple Displacement Amplification (MDA) amplified embryonic DNA was purified with AMPure XP beads (Beckman Coulter). Genomic DNA from the couple and purified embryonic MDA material was used for library preparation (Kapa Hyper Plus kit, Roche) and a targeted enrichment was performed using SeqCap EZ Human Exome v3.0 (Roche). The obtained library was sequenced on a Novaseq6000 (Illumina) generating 100bp paired-end reads. An average target coverage (exome) of 92.9x (maternal sample), 75.6x (paternal sample) and 71.2x (embryonic MDA amplified DNA) was obtained. Known polymorphic SNPs (with rs number) with at least 10 reads in the samples of the trio (for autosomes, X chromosome, PAR1 and PAR2) or at least 10 reads in the male parent (for Y chromosome) were selected for APCAD analysis. Results were compared with the results obtained with SNP array. For materials and methods with respect to the APCAD analysis using SNP array are described elsewhere (Example 1).

*Results*

The APCAD approach can be applied for genome-wide sequencing methods (whole genome, whole exome or other). Because BAF cannot be used (SNP array specific terminology), we introduced the term alternative allele frequency instead (ALAF): the percentage of reads with an alternative allele (non-reference) for a given SNP. Similar to the analysis of data obtained with SNP array, we focused again on the SNPs with a different homozygous genotype in both parents (subcategory 1A: SNPs for which the father is homozygous for the alternative allele and the mother is homozygous for the reference allele; subcategory 1B: SNPs for which the father is homozygous for the reference allele and mother is homozygous for the alternative allele) complemented with SNPs on chromosome Y (Table 10). Again, we calculated the difference between the means obtained for both subcategories of category 1 SNPs located on the same chromosome (delta_ALAF = mean subcategory 1B - mean subcategory 1A) with a minimal distance of 20kb. As a proof of principle, we compared the sequencing results obtained with results from SNP array for the same sample with a known meiotic trisomy 16 (Figure 4, Figure 8). Genome-wide 3097 SNPs were retained for analysis after sequencing compared to 11952 SNPs with SNP array. Despite the different numbers of available SNPs, similar results were obtained with both methods. With SNP array a delta_BAF of 0.285 was obtained for chromosome 16 based on 342 SNPs (153 of subcategory 1A and 189 of subcategory 1B), corresponding to a calculated %Mat of 66.42. With sequencing a delta_ALAF of 0.350 was obtained for chromosome 16 in this sample based on 116 SNPs (51 of subcategory 1A and 65 of subcategory 1B). If a linear (theoretical) relation between delta_ALAF and the maternal contribution is assumed, a delta_ALAF of 0.350 would correspond with a maternal contribution of 67.5% (delta_ALAF = 0.02 x %Mat - 1).

If a linear relation between allele frequency of maternal alleles of SNPs and maternal contribution exists, the maternal contribution can also be calculated in a more simplified way: by calculating the AF of maternal alleles for the selected category 1 SNPs and subsequently taking the mean of these AF values (or trimmed mean or median). For chromosome 16 of the sample with maternal trisomy16 the mean percentage of maternal alleles for the SNPs on chromosome 16 was 67.2% which is close to the theoretically expected 66.6% for a maternal trisomy. The mean can be weighed based on quality parameters (e.g. number of reads or square root of this value). In the simplest of approaches the number of maternal SNP alleles is counted for all SNPs in a region of interest and divided by the total number of alleles for those SNPs. For the example the resulting calculated maternal contribution is about the same (66.4%).

**Example 3: Use of segmentation algorithms for detection of segmental anomalies**

*Materials and Methods*

The R package GenWin v.0.1 uses cubic smoothing spline fitting to identify the appropriate window sizes. An advantage of using this approach over classic binning strategies is that the spline fitting determines windows of variable size. We combine adjacent windows if the direction of the anomaly remains the same. We then use a Wilcox test (R function wilcox.test) to determine whether or not the separation of the two groups is statistically significant. In order to call an interval as clinically relevant, we use a combination of p-value and either the difference of means of the two groups or alternative the difference of medians. We applied the cubic smoothing spline on an MDA amplified trophectoderm sample from an embryo diagnosed as unbalanced for the paternal reciprocal translocation t(1;10)(q41;q24) by haplotyping (Karyomapping).

*Results*

Application of a spline function on the category 1 SNPs identifies the unbalanced segments of the translocation: a shift compatible with a terminal deletion on chr1q (estimated paternal contribution ≈0%) and a smaller shift compatible with a paternal duplication on chromosome 10q (estimated paternal contribution ≈67%). The duplication is detected as two distinct duplications located in close proximity (Figure 9A). Optimization is needed further to determine the optimal parameters.

Homozygous SNP's that are compatible with Mendelian inheritance can be removed using a fixed cut-off (> 0.9 or < 0.1) for categories 2 and 3. E.g. in case the mother is AA and father is AB, an AA genotype is compatible with Mendelian inheritance. Now the spline function can also identify the unbalanced segments for category 2 and 3 SNPs (Figure 9B and 9C).

Another approach for category 2 and 3 SNPs is to analyze the ratio between heterozygous SNPs on one hand and homozygous SNPs of the expected genotype on the other hand. In case of random segregation in non-consanguineous couples, the groups of homozygous and heterozygous SNPs should be about equal in size. When there is a loss of homozygous SNPs for the category 2 SNPs this indicates the presence of both maternal haplotypes (e.g. maternal BPH trisomy or maternal UPD with heterodisomy). When there is a loss of homozygous SNPs for the category 3 SNPs this can indicate the presence of both paternal haplotypes (e.g. paternal BPH trisomy or paternal UPD with heterodisomy).

Alternatively, loss of homozygous SNPs of categories 2 or 3 can also be caused by consanguinity: in case the parents share a haplotype by common ancestry (identical by descent; IBD). In this case the SNPs of category 1 will be absent (except e.g. in case of genotyping errors). If a parent transmitted the IBD haplotype, all informative SNPs for this parent will be homozygous. If a parent transmits the non-IBD haplotype, all informative SNPS for this parent will be heterozygous (Figure 2).

**Example 4: Use of APCAD for mosaic aneuploidy detection in genomic DNA samples**

*Materials and Methods*

We selected two samples for analysis with APCAD. In one sample a marker chromosome was identified in 10 out of 35 metaphases (29%) by standard karyotyping but its origin had not been elucidated using BAC arrays. A second sample was selected because a mosaic trisomy 21 was detected with FISH on lymphocytes trisomy 12 out of 100 (12%) analyzed interphase nuclei.

*Results*

The example (Figure E3.1) shows a maternal mosaic 7.5Mb copy-number gain detected in an estimated 38% of cells (delta_BAF = 0.1328). This finding elucidated the origin of the small supernumerary marker chromosome (sSMC) present in this patient as a derivative chromosome 19. This was confirmed by metaphase and interphase FISH using locus specific chromosome 19 probes (data not shown).

In the patient with the low level mosaic trisomy 21 a higher maternal contribution was detected for chromosome 21 ($p < 0.001$ with the non-parametric Mann-Whitney U test) was observed (data not shown).

**Example 5: Alternative selection of AF frequencies for analysis**

Instead of using both subcategories for a given category, also a single subcategory can be used In this case, the allele is chosen for each SNP of a category that responds to the chosen subcategory. Theoretical examples are shown in Tables 11 and 12. Also any other combinations can be made (e.g. cat 1A + 1B and Cat 2A + 3B).

**Conclusion**

We have developed a new SNP based approach for aneuploidy detection in embryos that involves the measurement of the %Mat and hence also the paternal contribution (100- %Mat) for a chromosome in the sample. If the %Mat is skewed and the Log2R profile shows a shift for the chromosome or chromosome segment, the %Mat can be used to estimate the (minimal) percentage of aneuploid cells in the biopsy (Table 13). In this way samples with mosaic and non-mosaic aneuploidy can be discriminated, which is not possible using Karyomapping. This is useful as mosaic embryos have been reported to have a lower implantation rate and higher miscarriage rate but can lead to the birth of a healthy (or apparently healthy) child (Fragouli et al. 2017)(Greco, Minasi and Fiorentino 2015), (Munne et al. 2017), (Spinella et al. 2018, Victor et al. 2019), (Zhang et al. 2019, Zhang et al. 2020), (Zore et al. 2019), (Munné et al. 2020).

We conclude that the calculation of the maternal contribution provides a new useful universal tool for aneuploidy detection. It has the advantage that the maternal contribution can be measured without any assumptions. It is not influenced by the number of different maternal or paternal haplotypes present in the sample (e.g. SPH or BPH trisomy) or the location of recombination events.

Notably, the integration of the proposed parameters %Mat and %Pat in combination with accurate determination of copynumber (CN) by e.g. NGS will allow to determine the maternal copynumber (CNmat) and paternal copynumber (CNpat) for a chromosome or chromosome segment in the sample (CNmat =CN x %Mat/100; CNpat =CN x %Pat/100). This CNmat and CNPat have the potential to become an intuitive readout for aneuploidy detection in the future.

**References**

De Rycke, M., A. De Vos, F. Belva, V. Berckmoes, M. Bonduelle, A. Buysse, K. Keymolen, I. Liebaers, J. Nekkebroeck, P. Verdyck & W. Verpoest (2020) Preimplantation genetic testing with HLA matching: from counseling to birth and beyond. J Hum Genet.

Fragouli, E., S. Alfarawati, K. Spath, D. Babariya, N. Tarozzi, A. Borini & D. Wells (2017) Analysis of implantation and ongoing pregnancy rates following the transfer of mosaic diploid-aneuploid blastocysts. Hum Genet, 136, 805-819.

Greco, E., M. G. Minasi & F. Fiorentino (2015) Healthy Babies after Intrauterine Transfer of Mosaic Aneuploid Blastocysts. N Engl J Med, 373, 2089-90.

Handyside, A. H., G. L. Harton, B. Mariani, A. R. Thornhill, N. Affara, M. A. Shaw & D. K. Griffin (2010) Karyomapping: a universal method for genome wide analysis of genetic disease based on mapping crossovers between parental haplotypes. J Med Genet, 47, 651-8.

Kubicek, D., M. Hornak, J. Horak, R. Navratil, G. Tauwinklova, J. Rubes & K. Vesela (2019) Incidence and origin of meiotic whole and segmental chromosomal aneuploidies detected by karyomapping. Reprod Biomed Online, 38, 330-339.

Munne, S., J. Blazek, M. Large, P. A. Martinez-Ortiz, H. Nisson, E. Liu, N. Tarozzi, A. Borini, A. Becker, J. Zhang, S. Maxwell, J. Grifo, D. Babariya, D. Wells & E. Fragouli (2017) Detailed investigation into the cytogenetic constitution and pregnancy outcome of replacing mosaic blastocysts detected with the use of high-resolution next-generation sequencing. Fertil Steril, 108, 62-71.e8.

Munné, S., F. Spinella, J. Grifo, J. Zhang, M. P. Beltran, E. Fragouli & F. Fiorentino (2020) Clinical outcomes after the transfer of blastocysts characterized as mosaic by high resolution Next Generation Sequencing- further insights. Eur J Med Genet, 63, 103741.

Natesan, S. A., A. H. Handyside, A. R. Thornhill, C. S. Ottolini, K. Sage, M. C. Summers, M. Konstantinidis, D. Wells & D. K. Griffin (2014) Live birth after PGD with confirmation by a comprehensive approach (karyomapping) for simultaneous detection of monogenic and chromosomal disorders. Reprod Biomed Online, 29, 600-5.

Ottolini, C. S., S. Rogers, K. Sage, M. C. Summers, A. Capalbo, D. K. Griffin, J. Sarasa, D. Wells & A. H. Handyside (2015) Karyomapping identifies second polar body DNA persisting to the blastocyst stage: implications for embryo biopsy. Reprod Biomed Online, 31, 776-82.

Schinzel, A. 2001. Catalogue of unbalanced chromosome aberrations in man. Berlin ; New York: Walter de Gruyter.

Spinella, F., F. Fiorentino, A. Biricik, S. Bono, A. Ruberti, E. Cotroneo, M. Baldi, E. Cursio, M. G. Minasi & E. Greco (2018) Extent of chromosomal mosaicism influences the clinical outcome of in vitro fertilization treatments. Fertil Steril, 109, 77-83.

Victor, A. R., J. C. Tyndall, A. J. Brake, L. T. Lepkowsky, A. E. Murphy, D. K. Griffin, R. C. McCoy, F. L. Barnes, C. G. Zouves & M. Viotti (2019) One hundred mosaic embryos transferred prospectively in a single clinic: exploring when and why they result in healthy pregnancies. Fertil Steril, 111, 280-293.

Zamani Esteki, M., E. Dimitriadou, L. Mateiu, C. Melotte, N. Van der Aa, P. Kumar, R. Das, K. Theunis, J. Cheng, E. Legius, Y. Moreau, S. Debrock, T. D'Hooghe, P. Verdyck, M. De Rycke, K. Sermon, J. R. Vermeesch & T. Voet (2015) Concurrent whole-genome haplotyping and copy-number profiling of single cells. Am J Hum Genet, 96, 894-912.

Zhang, L., D. Wei, Y. Zhu, Y. Gao, J. Yan & Z. J. Chen (2019) Rates of live birth after mosaic embryo transfer compared with euploid embryo transfer. J Assist Reprod Genet, 36, 165-172.

Zhang, Y. X., J. J. Chen, S. Nabu, Q. S. Y. Yeung, Y. Li, J. H. Tan, W. Suksalak, S. Chanchamroen, W. Quangkananurug, P. S. Wong, J. P. W. Chung & K. W. Choy (2020) The Pregnancy Outcome of Mosaic Embryo Transfer: A Prospective Multicenter Study and Meta-Analysis. Genes (Basel), 11.

Zore, T., L. L. Kroener, C. Wang, L. Liu, R. Buyalos, G. Hubert & M. Shamonki (2019) Transfer of embryos with segmental mosaicism is associated with a significant reduction in live-birth rate. Fertil Steril, 111, 69-76.

**Table 1. Combinations of tubed cells with calculated percentage of maternal contribution (%Mat) for chromosome 21.**

| n cells 47,XY,+21 (mat) | n cells 46,XY | % cells with trisomy | n maternal chr21 copies | n paternal chr21 copies | % Maternal contribution (%Mat) |
|---|---|---|---|---|---|
| 0 | 8 | 0% | 8 | 8 | 8/16 (50%) |
| 1 | 7 | 12.5% | 9 | 8 | 9/17 (52.9%) |
| 2 | 6 | 25% | 10 | 8 | 10/18 (55.6%) |
| 3 | 5 | 37.5% | 11 | 8 | 11/19 (57.9%) |
| 4 | 4 | 50% | 12 | 8 | 12/20 (60.0%) |
| 5 | 3 | 62.5% | 13 | 8 | 13/21 (61.9%) |
| 6 | 2 | 75% | 14 | 8 | 14/22 (63.6%) |
| 7 | 1 | 87.5% | 15 | 8 | 15/23 (65.2%) |
| 8 | 0 | 100% | 16 | 8 | 16/24 (66.7%) |

## Table 2. The used categories and subcategories of SNPs after analysis with SNP array.

| | Paternal genotype | Maternal genotype | Expected genotype sample* | Allele frequency selected from | Remark |
|---|---|---|---|---|---|
| **Category 1** | | | | | |
| Subcategory 1A** | BB | AA | AB | B-allele (BAF) | |
| Subcategory 1B** | AA | BB | AB | B-allele (BAF) | |
| **Category 2** | | | | | |
| Subcategory 2A | BB | AB | AB or BB | B-allele (BAF) | Not phased |
| Subcategory 2B | AA | AB | AA or AB | B-allele (BAF) | Not phased |
| **Category 3** | | | | | |
| Subcategory 3A | AB | BB | AB or BB | B-allele (BAF) | Not phased |
| Subcategory 3B | AB | AA | AA or AB | B-allele (BAF) | Not phased |
| **Category 4** | | | | | |
| Subcategory 4A | BB | BB | BB | B-allele (BAF) | |
| Subcategory 4B | AA | AA | AA | B-allele (BAF) | |
| **Category 'other'** | | | | | |
| Other (with subcat. 1A) | BB | NC | NC or BB | B-allele (BAF) | Only for SNPs on Y chr. |
| Other (with subcat. 1B) | AA | NC | NC or AA | B-allele (BAF) | Only for SNPs on Y chr. |

*SNP genotype calls are written as AA, AB, BB or NC. "NC" indicates no call or very low signal intensity ($\log_2 R$ <-4). Hemizygous SNPs A/- and B/- are written as AA or BB respectively as hemizygosity and homozygosity cannot be distinguished.

## Table 3. Discordant results per chromosome regarding *de novo* non-mosaic chromosomal anomalies detected by analysis of Karyomapping and APCAD profiles.

| | Interpretation Karyomapping | Interpretation APCAD |
|---|---|---|
| 1 | BPH CN gain 17q21.34 to qter (2x mat) (A6) | BPH CN gain 17q21.34 to qter (3x mat) (~38Mb) (A6) |
| 2 | Mosaic monosomy chr10 (A1) | Pat deletion of 10q2.3 to 10q24.31 (~20Mb) and mosaic CN loss of the remainder of chr10 (A5)* |
| 3 | Mosaic monosomy chr3 (A1) | Pat deletion of pter to 3p24.2 (~25Mb) and mosaic CN loss of the remainder of chr3 (A5)* |
| 4 | Monosomy chr17 pat. (A6) | Pat deletion of 17q12 to qter (~49Mb) and mosaic CN loss of the remainder of chr17 (A6)* |
| 5 | SPH trisomy chr15 (A3) | Mosaic SPH trisomy chr15 (A1) |
| 6 | Uncertain chr14 (CN gain?) (A5) | SPH trisomy chr14 (A6) |

CN: copy number. SPH: single parental homolog; BPH: both parental homolog. Pat: paternal origin, Mat: maternal origin. Codes A1 to A6 for comparison with Table 6. *Low paternal contribution observed for the remainder of the chromosome indicates a limited proportion of cells within the biopsy with a derivative chromosome and a majority of cells with a monosomy (no paternal copy).

**Table 4. Concordant results per chromosome regarding *de novo* non-mosaic chromosomal anomalies detected by analysis of Karyomapping and APCAD profiles.**

| Type of anomaly | Mat. | Pat. | Remark |
|---|---|---|---|
| BPH trisomy autosome | 56 | 1 | |
| BPH trisomy XXY | 3 | 2 | |
| BPH segmental CN gain autosome | 1 | 0 | Sized 84Mb |
| BPH segmental CN gain and deletion on autosome | 1 | 0 | Isochromosome 9q (deletion of 9p and BPH copy number gain of 9q) |
| Monosomy autosome | 53 | 3 | |
| Monosomy X | 0 | 6 | |
| Deletion on autosome | 2 | 23 | Maternal: sized 13 and 84 Mb. Paternal: sized 10, 14, 19, 25, 27, 30, 31, 32, 34, 40, 43, 47, 50, 51, 65, 65, 70, 78, 78, 81, 93, 103 and 123 Mb. |
| Deletion on X | 1 | 0 | Sized 58 Mb. |
| SPH trisomy autosome | 8 | 4 | |
| SPH segmental CN gain autosome | 2 | 2 | Maternal: sized 15 and 39 Mb, paternal: sized 46 and 105 Mb. |

CN: copy number. SPH: single parental homolog; BPH: both parental homolog. Pat: paternal origin, Mat: Maternal origin.

**Table 5. Unbalanced inherited chromosomal anomalies detected by Karyomapping (PGT-SR approach) compared with analysis of APCAD profiles*.**

| Embryo | Parental karyotype | Segment size | Conclusion with Karyomapping (PGT-SR) | Conclusion with APCAD* |
|---|---|---|---|---|
| 1 | 45,XX,der(14;21)(q10;q10) | | Unbalanced | BPH trisomy 21 |
| | Segment 1: chr14q | 87Mb | Normal CN | Normal CN |
| | Segment 2: chr21q | 33Mb | CN gain (+1) | CN gain (+1) |
| 2 | 45,XX,der(14;21)(q10;q10) | | Unbalanced | BPH trisomy14, BPH tetrasomy 21 |
| | Segment 1: chr14q | 87Mb | CN gain (+1) | CN gain (+1) |
| | Segment 2: chr21q | 33Mb | CN gain (+2) | CN gain (+2) |
| 3 | 46,XX,t(14;17)(q32.3;q11.2) | | Unbalanced | Monosomy 14 |
| | Segment 1: chr14 centric | 77Mb | CN loss (-1) | CN loss (-1) |
| | Segment 2: chr14 transl. | 10Mb | CN loss (-1) | CN loss (-1) |
| | Segment 3: chr17 centric | 29Mb | Normal CN | Normal CN |
| | Segment 4: chr17 transl. | 52Mb | Normal CN | Normal CN |
| 4 | 46,XX,t(14;17)(q32.3;q11.2) | | Unbalanced | BPH trisomy 14, deletion on chr17 |
| | Segment 1: chr14 centric | 77Mb | CN gain (+1) | CN gain (+1) |
| | Segment 2: chr14 transl. | 10Mb | Normal CN** | CN gain (+1)** |
| | Segment 3: chr17 centric | 29Mb | CN loss (-1) | CN loss (-1) |
| | Segment 4: chr17 transl. | 52Mb | Normal CN | Normal CN |
| 5 | 46,XX,t(6;15)(q25.3;q11.1) | | Unbalanced | Deletion on chr6q, CN gain on 15q |
| | Segment 1: chr6 centric | 160Mb | Normal CN | Normal CN |
| | Segment 2: chr6 transl. | 11Mb | CN loss (-1) | CN loss (-1) |
| | Segment 3: chr15 centric | 24Mb | Normal CN | Normal CN |
| | Segment 4: chr15 transl. | 58Mb | CN gain (+1) | CN gain (+1) |

*Analysis of APCAD profiles was performed without knowledge of the indication. **One segment of 10Mb with normal copy number was not detected by analyzing APCAD profiles. BPH: both parental homolog; CN: Copy number.

EP 4 118 652 B1

## Table 6. Comparison of results per embryo for detection of *de novo* and inherited non-mosaic and BPH chromosomal anomalies.

|  | Meiotic or non-mosaic anomaly detected | Karyomapping (rBAF, Log$_2$R and haplotypes) | APCAD (rBAF, Log$_2$R and cBAF) |
|---|---|---|---|
| A1 | None | 208 | 207 |
| A2 | Single BPH trisomy | 38 | 38 |
| A3 | Single SPH trisomy | 4 | 3 |
| A4 | Single monosomy | 44 | 44 |
| A5 | Single segmental anomaly | 26 | 27 |
| A6 | Combined anomalies | 29 | 30 |
|  | Sum | 349 | 349 |

## Table 7. Maternal contribution observed for autosomes split by the type of chromosome anomaly detected by analysis of APCAD profiles (*de novo* anomalies) and Karyomapping (inherited anomalies)

| Measured %Mat | | Chromosome anomaly | | | | | | | |
|---|---|---|---|---|---|---|---|---|---|
| lower | upper | Tetra-somy | BPH trisomy | (mosaic) SPH trisomy | Mosaic mono-somy | (Mosaic) Seg-mental | Mono-somy | N.A.D. | Total |
| ≥0.0% | <25.0% | 0 | 0 | 0 | 4 | 1 | 54 | 0 | 59 |
| ≥25.0% | <36.4% | 0 | 1 | 4 | 9 | 2 | 0 | 14 | 30 |
| ≥36.4% | <44.4% | 0 | 0 | 5 | 8 | 3 | 0 | 117 | 133 |
| ≥44.4% | ≤55.6% | 0 | 1 | 1 | 3 | 12 | 0 | 7136 | 7153 |
| >55.6% | ≤63.6% | 0 | 1 | 4 | 5 | 23 | 0 | 151 | 184 |
| >63.6% | ≤75.0% | 1 | 55 | 9 | 5 | 9 | 0 | 18 | 97 |
| >75.0% | ≤100% | 0 | 1 | 0 | 4 | 14 | 3 | 0 | 22 |
| Total | | 1 | 59 | 23 | 38 | 64 | 57 | 7436 | 7678 |

N.A.D.: no anomalies detected for this chromosome; BPH: both parental homolog; %Mat: maternal contribution.

26

**Table 8. Maternal contribution observed for sex chromosomes split by the type of chromosome anomaly detected by analysis of APCAD profiles**

| Measured %Mat | | Chromosome anomaly | | | | | | Total |
|---|---|---|---|---|---|---|---|---|
| lower | upper | X in female | X in male | Mono-somy X | X in XXY | Y present | Y Absent | |
| ≥0.0% | <25.0% | 0 | 0 | 0 | 0 | 173 | 0 | 173 |
| ≥25.0% | <36.4% | 0 | 0 | 0 | 0 | 0 | 0 | 0 |
| ≥36.4% | <44.4% | 3 | 0 | 0 | 0 | 0 | 0 | 3 |
| ≥44.4% | ≤55.6% | 161 | 0 | 0 | 2 | 0 | (176) | 163 (339) |
| >55.6% | ≤63.6% | 3 | 0 | 0 | 0 | 0 | 0 | 3 |
| >63.6% | ≤75.0% | 0 | 0 | 0 | 0 | 0 | 0 | 0 |
| >75.0% | ≤100% | 0 | 168 | 6 | 3 | 0 | 0 | 168 |
| Total | | 167 | 168 | 6 | 5 | 173 | 176 | |

N.A.D.: no anomalies detected for this chromosome; BPH: both parental homolog; %Mat: maternal contribution.

**Table 9. Maternal contribution observed for pseudo-autosomal regions split by the type of chromosome anomaly detected by analysis of APCAD profiles**

| Measured %Mat | | Chromosome anomaly | | | | | | Total |
|---|---|---|---|---|---|---|---|---|
| lower | upper | Segmental X | Mosaic monosomy | Monosomy X | XXY pat | XXY mat | N.A.D. | |
| ≥0.0% | <25.0% | 1 | 0 | 0 | 0 | 0 | 0 | 1 |
| ≥25.0% | <36.4% | 0 | 1 | 0 | 2 | 0 | 3 | 6 |
| ≥36.4% | <44.4% | 0 | 0 | 0 | 0 | 0 | 31 | 31 |
| ≥44.4% | ≤55.6% | 0 | 0 | 0 | 0 | 0 | 251 | 251 |
| >55.6% | ≤63.6% | 0 | 2 | 0 | 0 | 0 | 46 | 48 |
| >63.6% | ≤75.0% | 0 | 0 | 0 | 0 | 3 | 1 | 4 |
| >75.0% | ≤100% | 0 | 0 | 6 | 0 | 0 | 0 | 6 |
| Not calculated | | 0 | 0 | 0 | 0 | 0 | 2 | 2 |
| | | 1 | 3 | 6 | 2 | 3 | 334 | 349 |

N.A.D.: no anomalies detected for this chromosome; BPH: both parental homolog; %Mat: maternal contribution.

## Table 10. The used categories and subcategories of SNPs after analysis with sequencing.

| | Paternal genotype* | Maternal genotype * | Expected genotype sample* | Allele frequency selected from | Remark |
|---|---|---|---|---|---|
| **Category 1** | | | | | |
| Subcategory 1A | 1/1 | 0/0 | 0/1 | Alt. allele (ALAF) | |
| Subcategory 1B | 0/0 | 1/1 | 0/1 | Alt. allele (ALAF) | |
| **Category 2** | | | | | |
| Subcategory 2A | 1/1 | 0/1 | 0/1 or 1/1 | Alt. allele (ALAF) | Not phased |
| Subcategory 2B | 0/0 | 0/1 | 0/0 or 0/1 | Alt. allele (ALAF) | Not phased |
| **Category 3** | | | | | |
| Subcategory 3A | 0/1 | 1/1 | 0/1 or 1/1 | Alt. allele (ALAF) | Not phased |
| Subcategory 3B | 0/1 | 0/0 | 0/0 or 0/1 | Alt. allele (ALAF) | Not phased |
| **Category 4** | | | | | |
| Subcategory 4A | 1/1 | 1/1 | 1/1 | Alt. allele (ALAF) | |
| Subcategory 4B | 0/0 | 0/0 | 0/0 | Alt. allele (ALAF) | |
| **Category 'other'** | | | | | |
| Other (with subcat. 1A) | 1/1 | ./. | ./. or 1/1 | Alt. allele (ALAF) | Only for SNPs on Y chr |

*SNP genotypes calls are written as 0/0 (homozygous for the allele in the reference genome GRCh37/hg19), 0/1 (heterozygous), 1/1 (homozygous alternative allele) or "./." (absence of reads). 'ALAF': alternative allele frequency. Hemizygous SNPs 1/- and 0/- are written as 1/1 or 0/0 respectively as hemizygosity and homozygosity cannot be distinguished. Alt.: alternative (non-reference).

## Table 11. Example of an alternative approach 1

| | Paternal genotype* | Maternal genotype* | Expected genotype sample* | Allele frequency selected from | Remark |
|---|---|---|---|---|---|
| Category 1A | AA | BB | AB | A-allele (AAF) | |
| | BB | AA | AB | B-allele (BAF) | |
| Category 2A | AA | AB | AA or AB | A-allele (AAF) | Not phased |
| | BB | AB | AB or BB | B-allele (BAF) | Not phased |
| Category 3A | AB | AA | AA or AB | A-allele (AAF) | Not phased |
| | AB | BB | AB or BB | B-allele (BAF) | Not phased |
| Other** (combined with cat 1A) | AA | NC | NC or AA | A-allele (AAF) | Only for SNPs on Y chr |
| | BB | NC | NC or BB | B-allele (BAF) | Only for SNPs on Y chr |

*SNP genotype calls are written as AA, AB, BB or NC. "NC" indicates no call or very low signal intensity ($\log_2 R < -4$). Hemizygous SNPs A/- and B/- are written as AA or BB respectively as hemizygosity and homozygosity cannot be distinguished.

## Table 12. Example of an alternative approach 2

|  | Paternal genotype* | Maternal genotype* | Expected genotype sample* | Allele frequency selected from | Remark |
|---|---|---|---|---|---|
| Category 1B | AA | BB | AB | B-allele (BAF) | |
| | BB | AA | AB | A-allele (AAF) | |
| Category 2B | AA | AB | AA or AB | B-allele (BAF) | Not phased |
| | BB | AB | AB or BB | A-allele (AAF) | Not phased |
| Category 3B | AB | AA | AA or AB | B-allele (BAF) | Not phased |
| | AB | BB | AB or BB | A-allele (AAF) | Not phased |
| Other (combined with cat 1B) | AA | NC | NC or AA | B-allele (BAF) | Only for SNPs on Y chr |
| | BB | NC | NC or BB | A-allele (AAF) | Only for SNPs on Y chr |

*SNP genotype calls are written as AA, AB, BB or NC. "NC" indicates no call or very low signal intensity ($\log_2 R < -4$). Hemizygous SNPs A/- and B/- are written as AA or BB respectively as hemizygosity and homozygosity cannot be distinguished.

**Table 13. Relation between %Mat and predicted percentage of aneuploid cells for mosaic monosomy (normal disomy/monosomy) and mosaic trisomy (normal disomy/trisomy).**

| %Mat | Theoretical Delta_BAF (NGS£) | Delta_BAF Empirical (SNP array) | % monosomic cells if mosaic monosomy / disomy# | % trisomic cells if mosaic trisomy / disomy# |
|---|---|---|---|---|
| 0.0 | -1.000 | -0.978 | 100.0%$ | 0% (100%UPD pat) |
| 11.1 | -0.778 | -0.732 | 87.5%$ | 25% (75%UPD pat) |
| 20.0 | -0.600 | -0.547 | **75.0%$** | 50% (50% UPD pat) |
| 27.3 | -0.455 | -0.404 | 62.5%$ | 75.0% (25% UPD pat) |
| 33.3 | -0.333 | -0.289 | 50.0%$ | 100.0%* |
| 38.5 | -0.231 | -0.196 | 37.5%$ | 60.0%* |
| 42.9 | -0.143 | -0.119 | **25.0%$** | 33.3%* |
| 46.7 | -0.067 | -0.055 | 12.5%$ | 14.3%* |
| 50.0 | 0.000 | 0.000 | 0.0% | 0.0% |
| 53.3 | 0.067 | 0.055 | 12.5%* | 14.3%$ |
| 57.1 | 0.143 | 0.119 | **25.0%*** | 33.3%$ |
| 61.5 | 0.231 | 0.196 | 37.5%* | 60.0%$ |
| 66.7 | 0.333 | 0.289 | 50.0%* | 100.0%$ |
| 72.7 | 0.455 | 0.404 | 62.5%* | 75.0% (25% UPD mat) |
| 80.0 | 0.600 | 0.547 | **75.0%*** | 50% (50% UPD mat) |
| 88.9 | 0.778 | 0.732 | 87.5%* | 25% (75% UPD mat) |
| 100.0 | 1.000 | 0.978 | 100.0%* | 0% (100% UPD mat) |
| 33.3 | -0.333 | -0.289 | 50.0%$ | 100.0%* |
| 34.8 | -0.304 | -0.263 | 46.7%$ | 87.5%* |
| 36.4 | -0.273 | -0.234 | 42.9%$ | **75.0%*** |
| 38.1 | -0.238 | -0.203 | 38.5%$ | 62.5%* |
| 40.0 | -0.200 | -0.169 | 33.3%$ | 50.0%* |
| 42.1 | -0.158 | -0.132 | 27.3%$ | 37.5%* |
| 44.4 | -0.111 | -0.092 | 20.0%$ | **25.0%*** |
| 47.1 | -0.059 | -0.048 | 11.1%$ | 12.5%* |
| 50.0 | 0.000 | 0.000 | 0.0% | 0.0% |
| 52.9 | 0.059 | 0.048 | 11.1%* | 12.5%$ |
| 55.6 | 0.111 | 0.092 | 20.0%* | **25.0%$** |
| 57.9 | 0.158 | 0.132 | 27.3%* | 37.5%$ |
| 60.0 | 0.200 | 0.169 | 33.3%* | 50.0%$ |
| 61.9 | 0.238 | 0.203 | 38.5%* | 62.5%$ |
| 63.6 | 0.273 | 0.234 | 42.9%* | **75.0%$** |
| 65.2 | 0.304 | 0.263 | 46.7%* | 87.5%$ |
| 66.7 | 0.333 | 0.289 | 50.0%* | 100.0%$ |

*loss (mosaic monosomy) or gain (mosaic trisomy) of the paternal copy.
$loss (mosaic monosomy) or gain (mosaic trisomy) of the maternal copy.
#normal disomy, not UPD unless stated otherwise
£with NGS the delta_BAF is to be close to the theoretical delta_BAF.

**Claims**

1. A computer implemented method for the analysis of genetic material in a subject, said method comprising:

   - obtaining unphased genotype information of polymorphic variants of a first and second parent of the subject;
   - obtaining the genomic location of the polymorphic variants;
   - selection of the polymorphic variants based on one or more of the following criteria:

     - polymorphic variants for which the first and second parent are homozygous or hemizygous for a different allele (category 1 polymorphic variants);
     - polymorphic variants for which the first parent is homozygous for a specific allele and the second parent is heterozygous for said specific allele (category 2 polymorphic variants); or
     - polymorphic variants for which the second parent is homozygous for a specific allele and the first parent is heterozygous for said specific allele (category 3 polymorphic variants);

   - obtaining the allele frequency (AF) values for said selected polymorphic variants in genetic material of the subject;
   - selection of one allele per polymorphic variant and subcategorization of its corresponding AF frequency of the subject in one of the following subcategories:

     - AF values of the category 1 polymorphic variants, representing the AF values for alleles present in homozygous or hemizygous state in the first parent (subcategory 1A);
     - AF values of the category 1 polymorphic variants, representing the AF values for alleles present in homozygous or hemizygous state in the second parent (subcategory 1B);
     - AF values of the category 2 polymorphic variants, representing the AF values for alleles present in homozygous or hemizygous state in the first parent (subcategory 2A);
     - AF values of the category 2 polymorphic variants, representing the AF values for alleles heterozygous in the second parent and absent in the first parent (subcategory 2B);
     - AF values of the category 3 polymorphic variants, representing the AF values for alleles present in homozygous or hemizygous state in the second parent (subcategory 3A); or
     - AF values of the category 3 polymorphic variants, representing the AF values for alleles heterozygous in the first parent and absent in the second parent (subcategory 3B);

   - calculation of the mean AF values, the trimmed mean AF values or the median AF values of the polymorphic variants for each of the given subcategories, wherein the polymorphic variants are located between two genomic locations on a chromosome and evaluating whether a genetic anomaly is present in the genetic material of the subject based on the AF values of the polymorphic variants in one or more of the subcategories and the genomic location of said polymorphic variants; and wherein a genetic anomaly is present in the genetic material of the subject when the AF values deviates from 0.5; in particular when the AF value deviates from 0.5 with a value of at least and about 0.025 ; more in particular when the AF value deviates from 0.5 with a value of at least and about 0.045.

2. The method according to claim 1, further comprising;

   - calculation of the difference between the median AF values, the mean AF values or the trimmed mean AF values of subcategories 1A and 1B or between the median AF values, the mean AF values or the trimmed mean AF values of subcategories 2A and 2B, or between the median AF values, the mean AF values or the trimmed mean AF values of subcategories 3A and 3B said difference being indicated as 'delta AF';
   - evaluating whether a genetic anomaly is present in the genetic material of the subject based on the 'delta AF' values observed between said genomic locations, and wherein a genetic anomaly is present when the delta AF value deviates from 0; in particular when the delta AF value deviates from 0 with a value of at least and about 0.05; more in particular when the delta AF value deviates from 0 with a value of at least and about 0.09.

3. The method according to claims 1 or 2 wherein polymorphic variants or SNPs that are distributed less than 50 kb, preferably less than 20 kb from each other are removed from further analysis.

4. The method according to any of the preceding claims wherein delta AF values are used to calculate a value for the parental contribution between said genomic locations and evaluating whether a genetic anomaly is present in the genetic material of the subject based on said value for parental contribution observed between said genomic locations.

5. The method according to claim 4, wherein calculating a value for the parental contribution between said genomic locations, and in particular a value for the percentage parental contribution (%Mat or %Pat), is based on a second order generalized linear model between the delta AF values and the percentage parental contribution (%Mat or %Pat) across said genomic locations; and wherein a parental contribution deviating from 50%; in particular a deviation of at least and about 3% is indicative for a chromosomal anomaly.

6. The method according to claim 4, wherein a %Mat or %Pat between and about 44.4% and 55.6% is indicative for a normal disomy; wherein a %Mat ot %Pat between and about 63.6% and 72.7% is indicative for a trisomy; and wherein a %Mat or %Pat between and about 0% and 3.3%, is indicative for a monosomy.

7. The method according to any of the preceding claims wherein the selected allele per polymorphic variant is an allele with a specific feature, said feature selected from the A allele, the B allele, the allele with the higher allele frequency in a given population, the allele with the lower allele frequency in a given population, a reference allele in a given reference genome, the allele present in homozygous state in parent 1, the allele present in homozygous state in parent 2, the allele present in heterozygous state in parent 1 but absent in parent 2, or the allele present in heterozygous state in parent 2 but absent in parent 1; preferably wherein the selected allele per polymorphic variant is the B allele; even more preferably wherein the selected allele per polymorphic variant is the B allele comprising a single nucleotide polymorphism (SNP).

8. The method according to any of the preceding claims wherein the selected AF values of the polymorphic variants of the subcategories 2A, 2B, 3A and/or 3B are converted into discrete genotype calls and wherein it is evaluated whether homozygous or heterozygous allele frequency values are underrepresented or overrepresented between two particular genomic locations.

9. The method according to any of the preceding claims wherein the genetic material of the subject is isolated from a sample comprising a low amount of genetic material of said subject; such as a sample comprising only one or few cells of said subject; or a plasma sample obtained from a mother pregnant with said subject.

10. The method of any of the preceding claims, wherein the genetic anomaly comprises a numerical or structural chromosomal abnormality present in all (non-mosaic) or only a part of the biopsied cells (mosaic); in particular a numerical or structural chromosomal abnormality selected from a monosomy, uniparental disomy, trisomy, tetrasomy, a duplication, a deletion, respectively a mosaic monosomy, mosaic disomy, mosaic trisomy, mosaic tetrasomy, a mosaic tandem duplication, a mosaic deletion and combinations thereof.

11. The method according to claim 10, wherein when the delta AF value deviates from 0, below the threshold of about 0.09 (in particular below 0.0924) the value is considered normal (normal disomy); when the delta AF value deviates from 0, above a threshold of about 0.2 (in particular above 0.234) and an increased copynumber is observed, the value indicates a full trisomy or duplication; and when the delta AF value deviates from 0, between both threshold values and an increased copy number is observed, the sample is categorized as mosaic trisomy/disomy or mosaic duplication.

12. The method according to claim 10, wherein when the delta AF value deviates from 0, below the threshold of about 0.09 (in particular below 0.0924) the value is considered normal (normal disomy); when the delta AF value deviates from 0 above a threshold of about 0.9 and a decreased copynumber is observed, the value indicates a full monosomy or deletion; and when the delta AF value deviates from 0, between both threshold values and an decreased copynumber is observed the sample is categorized as mosaic monosomy/disomy or mosaic deletion.

13. The method of any of the preceding claims, wherein the polymorphic variants are selected from single nucleotide polymorphisms (SNPs), short tandem repeats (STRs); preferably selected from single nucleotide polymorphisms.

14. A computer program product which is capable, when executed on a processing engine, to perform the method of any of the preceding claims.

15. A non-transitory machine-readable storage medium storing the computer program product of claim 14.

**Patentansprüche**

1. Computerimplementiertes Verfahren für die Analyse von genetischem Material in einem Subjekt, das Verfahren umfassend:

    - Erhalten von nicht phasierten Genotyp-Informationen polymorpher Varianten eines ersten und zweiten Elternteils des Subjekts;
    - Erhalten des Genlocus der polymorphen Varianten;
    - Auswahl der polymorphen Varianten basierend auf einem oder mehreren der folgenden Kriterien:

        - polymorphe Varianten, bei denen der erste und der zweite Elternteil homozygot oder hemizygot für ein anderes Allel sind (polymorphe Varianten der Kategorie 1);
        - polymorphe Varianten, bei denen der erste Elternteil homozygot für ein spezifisches Allel und der zweite Elternteil heterozygot für dieses spezifische Allel ist (polymorphe Varianten der Kategorie 2); oder
        - polymorphe Varianten, bei denen der zweite Elternteil homozygot für ein spezifisches Allel und der erste Elternteil heterozygot für dieses spezifische Allel ist (polymorphe Varianten der Kategorie 3);

            - Erhalten der Allelfrequenzwerte (AF) für die ausgewählten polymorphen Varianten in dem genetischen Material des Subjekts;

    - Auswahl eines Allels pro polymorpher Variante und Unterkategorisierung der entsprechenden AF-Häufigkeit des Subjekts in eine der folgenden Unterkategorien:

        - AF-Werte der polymorphen Varianten der Kategorie 1, die die AF-Werte für Allele darstellen, die in dem ersten Elternteil in einem homozygoten oder hemizygoten Zustand vorhanden sind (Unterkategorie 1A);
        - AF-Werte der polymorphen Varianten der Kategorie 1, die die AF-Werte für Allele darstellen, die in dem zweiten Elternteil in dem homozygoten oder hemizygoten Zustand vorhanden sind (Unterkategorie 1B);
        - AF-Werte der polymorphen Varianten der Kategorie 2, die die AF-Werte für Allele darstellen, die in dem ersten Elternteil in dem homozygoten oder hemizygoten Zustand vorhanden sind (Unterkategorie 2A);
        - AF-Werte der polymorphen Varianten der Kategorie 2, die die AF-Werte für Allele darstellen, die in dem zweiten Elternteil heterozygot und in dem ersten Elternteil nicht vorhanden sind (Unterkategorie 2B);
        - AF-Werte der polymorphen Varianten der Kategorie 3, die die AF-Werte für Allele darstellen, die in dem zweiten Elternteil in dem homozygoten oder hemizygoten Zustand vorhanden sind (Unterkategorie 3A); oder
        - AF-Werte der polymorphen Varianten der Kategorie 3, die die AF-Werte für Allele darstellen, die in dem ersten Elternteil heterozygot und in dem zweiten Elternteil nicht vorhanden sind (Unterkategorie 3B);
        - Berechnung der mittleren AF-Werte, der getrimmten mittleren AF-Werte oder der Median-AF-Werte der polymorphen Varianten für jede der gegebenen Unterkategorien, wobei sich die polymorphen Varianten zwischen zwei Genloci auf einem Chromosom befinden, und Bewerten, ob in dem genetischen Material des Subjekts eine genetische Anomalie vorhanden ist, basierend auf den AF-Werten der polymorphen Varianten in einer oder mehreren der Unterkategorien und des Genlocus der polymorphen Varianten; und wobei eine genetische Anomalie in dem genetischen Material des Subjekts vorhanden ist, wenn die AF-Werte von 0.5 abweichen; speziell, wenn der AF-Wert mit einem Wert von mindestens und etwa 0.025 von 0.5 abweicht; noch spezieller, wenn der AF-Wert mit einem Wert von mindestens und etwa 0.045 von 0.5 abweicht.

2. Verfahren nach Anspruch 1, ferner umfassend:

    - Berechnung der Differenz zwischen den Median-AF-Werten, den mittleren AF-Werten oder den getrimmten mittleren AF-Werten der Unterkategorien 1A und 1B oder zwischen den Median-AF-Werten, den mittleren AF-Werten oder den getrimmten mittleren AF-Werten der Unterkategorien 2A und 2B oder zwischen den Median-AF-Werten, den mittleren AF-Werten oder den getrimmten mittleren AF-Werten der Unterkategorien 3A und 3B, wobei diese Differenz als "Delta-AF" bezeichnet wird;
    - Bewerten, ob in dem genetischen Material des Subjekts eine genetische Anomalie vorhanden ist, basierend auf den "Delta-AF"-Werten, die zwischen den Genloci beobachtet werden, und wobei eine genetische Anomalie vorhanden ist, wenn der Delta-AF-Wert von 0 abweicht; speziell, wenn der Delta-AF-Wert mit einem Wert von mindestens und etwa 0.05 von 0 abweicht; noch spezieller, wenn der Delta-AF-Wert mit einem Wert von mindestens du etwa 0.09 von 0 abweicht.

3. Verfahren nach Anspruch 1 oder 2, wobei polymorphe Varianten oder SNPs, die weniger als 50 kb, vorzugsweise weniger als 20 kb voneinander entfernt sind, aus der weiteren Analyse entfernt werden.

4. Verfahren nach einem der vorstehenden Ansprüche, wobei Delta-AF-Werte verwendet werden, um einen Wert für den elterlichen Beitrag zwischen den Genloci zu berechnen und zu bewerten, ob in dem genetischen Material des Subjekts eine genetische Anomalie vorhanden ist, basierend auf dem Wert für den elterlichen Beitrag, der zwischen den Genloci beobachtet wird.

5. Verfahren nach Anspruch 4, wobei das Berechnen eines Werts für den elterlichen Beitrag zwischen den Genloci und speziell eines Werts für den prozentualen elterlichen Beitrag (%Mat oder %Pat) auf einem verallgemeinerten linearen Modell zweiter Ordnung zwischen den Delta-AF-Werten und dem prozentualen elterlichen Beitrag (%Mat oder %Pat) über die Genloci hinweg basiert; und wobei ein von 50 % abweichender elterlicher Beitrag; noch spezieller eine Abweichung von mindestens und etwa 3 %, auf eine Chromosomenanomalie hinweist.

6. Verfahren nach Anspruch 4, wobei ein %Mat oder %Pat zwischen etwa 44,4 % und 55,6 % auf eine normale Disomie hinweist; wobei ein %Mat oder %Pat zwischen etwa 63,6 % und 72,7 % auf eine Trisomie hinweist; und wobei ein % Mat oder %Pat zwischen etwa 0 % und 3,3 % auf eine Monosomie hinweist.

7. Verfahren nach einem der vorstehenden Ansprüche, wobei das ausgewählte Allel pro polymorpher Variante ein Allel mit einem spezifischen Merkmal ist, wobei das Merkmal aus dem A-Allel, dem B-Allel, dem Allel mit der höheren Allelfrequenz in einer gegebenen Population, dem Allel mit der niedrigeren Allelfrequenz in einer gegebenen Population, einem Referenzallel in einem gegebenen Referenzgenom, dem in Elternteil 1 in dem homozygoten Zustand vorhandenen Allel, dem in Elternteil 2 in dem homozygoten Zustand vorhandenen Allel, dem in Elternteil 1 in dem heterozygoten Zustand vorhandenen, aber in Elternteil 2 fehlenden Allel oder dem in Elternteil 2 in dem heterozygoten Zustand vorhandenen, aber in Elternteil 1 fehlenden Allel ausgewählt ist; vorzugsweise wobei das ausgewählte Allel pro polymorpher Variante das B-Allel ist; noch mehr bevorzugt, wobei das ausgewählte Allel pro polymorpher Variante das B-Allel ist, umfassend einen Einzelnukleotid-Polymorphismus (SNP).

8. Verfahren nach einem der vorstehenden Ansprüche, wobei die ausgewählten AF-Werte der polymorphen Varianten der Unterkategorien 2A, 2B, 3A und/oder 3B in diskrete Genotyp-Calls umgewandelt werden und wobei ausgewertet wird, ob homozygote oder heterozygote Allelfrequenzwerte zwischen zwei speziellen Genloci unterrepräsentiert oder überrepräsentiert sind.

9. Verfahren nach einem der vorstehenden Ansprüche, wobei das genetische Material des Subjekts aus einer Probe isoliert wird, umfassend eine geringe Menge an genetischem Material des Subjekts; wie eine Probe, umfassend nur eine oder wenige Zellen des Subjekts; oder eine Plasmaprobe, die von einer mit dem Subjekt schwangeren Mutter gewonnen wurde.

10. Verfahren nach einem der vorstehenden Ansprüche, wobei die genetische Anomalie eine numerische oder strukturelle Chromosomenanomalie umfasst, die in allen (nicht-mosaikartigen) oder nur in einem Teil der biopsierten Zellen (Mosaik) vorhanden ist; speziell eine numerische oder strukturelle Chromosomenanomalie, die aus einer Monosomie, uniparentalen Disomie, Trisomie, Tetrasomie, einer Duplikation, einer Deletion beziehungsweise einer Mosaik-Monosomie, Mosaik-Disomie, Mosaik-Trisomie, Mosaik-Tetrasomie, einer Mosaik-Tandem-Duplikation, einer Mosaik-Deletion und Kombinationen davon ausgewählt ist.

11. Verfahren nach Anspruch 10, wobei, wenn der Delta-AF-Wert von 0 abweicht, unter den Schwellenwert von etwa 0.09 (speziell unter 0.0924), der Wert als normal angesehen wird (normale Disomie); wenn der Delta-AF-Wert von 0 abweicht, über einen Schwellenwert von etwa 0.2 (speziell über 0.234) und eine erhöhte Kopienzahl beobachtet wird, der Wert auf eine vollständige Trisomie oder Duplikation hinweist; und wenn der Delta-AF-Wert zwischen beiden Schwellenwerten von 0 abweicht und eine erhöhte Kopienzahl beobachtet wird, die Probe als Mosaik-Trisomie/Disomie oder Mosaik-Duplikation kategorisiert wird.

12. Verfahren nach Anspruch 10, wobei, wenn der Delta-AF-Wert von 0 abweicht, unter den Schwellenwert von etwa 0.09 (speziell unter 0.0924), der Wert als normal angesehen wird (normale Disomie); wenn der Delta-AF-Wert über einen Schwellenwert von etwa 0.9 von 0 abweicht und eine verringerte Kopienzahl beobachtet wird, der Wert auf eine vollständige Monosomie oder Deletion hinweist; und wenn der Delta-AF-Wert zwischen beiden Schwellenwerten von 0 abweicht und eine verringerte Kopienzahl beobachtet wird, die Probe als Mosaik-Monosomie/Disomie oder Mosaik-Deletion kategorisiert wird.

**13.** Verfahren nach einem der vorstehenden Ansprüche, wobei die polymorphen Varianten aus Einzelnukleotid-Polymorphismen (SNPs), kurzen Tandem-Wiederholungen (STRs) ausgewählt sind; vorzugsweise ausgewählt aus Einzelnukleotid-Polymorphismen.

**14.** Computerprogrammprodukt, das bei Ausführung auf einer Verarbeitungsmaschine in der Lage ist, das Verfahren nach einem der vorstehenden Ansprüche durchzuführen.

**15.** Nicht-transitorisches, maschinenlesbares Speichermedium, das das Computerprogrammprodukt nach Anspruch 14 speichert.

**Revendications**

**1.** Procédé implémenté par ordinateur destiné à l'analyse de matériel génétique chez un sujet, ledit procédé comprenant :

- l'obtention d'informations génotypiques non phasées de variants polymorphiques d'un premier et d'un second parent du sujet ;
- l'obtention de la localisation génomique des variants polymorphiques ;
- la sélection des variants polymorphiques en fonction d'un ou plusieurs des critères suivants :

- des variants polymorphiques pour lesquels le premier et le second parent sont homozygotes ou hémizygotes pour un allèle différent (variants polymorphiques de catégorie 1) ;
- des variants polymorphiques pour lesquels le premier parent est homozygote pour un allèle spécifique et le second parent est hétérozygote pour ledit allèle spécifique (variants polymorphiques de catégorie 2) ; ou
- variants polymorphiques pour lesquels le second parent est homozygote pour un allèle spécifique et le premier parent est hétérozygote pour ledit allèle spécifique (variants polymorphiques de catégorie 3) ;
- l'obtention des valeurs de fréquence allélique (AF) pour lesdits variants polymorphiques sélectionnés dans du matériel génétique du sujet ;
- sélection d'un allèle par variant polymorphique et sous-catégorisation de sa fréquence AF correspondante du sujet dans l'une des sous-catégories suivantes :

- valeurs AF des variants polymorphiques de catégorie 1, représentant les valeurs AF pour des allèles présents dans un état homozygote ou hémizygote chez le premier parent (sous-catégorie 1A) ;
- valeurs AF des variants polymorphiques de catégorie 1, représentant les valeurs AF pour des allèles présents dans l'état homozygote ou hémizygote chez le second parent (sous-catégorie 1B) ;
- valeurs AF des variants polymorphiques de catégorie 2, représentant les valeurs AF pour des allèles présents dans un état homozygote ou hémizygote chez le premier parent (sous-catégorie 2A) ;
- valeurs AF des variants polymorphiques de catégorie 2, représentant les valeurs AF pour des allèles hétérozygotes chez le second parent et absents chez le premier parent (sous-catégorie 2B) ;
- valeurs AF des variants polymorphiques de catégorie 3, représentant les valeurs AF pour des allèles présents dans un état homozygote ou hémizygote chez le second parent (sous-catégorie 3A) ; ou
- valeurs AF des variants polymorphiques de catégorie 3, représentant les valeurs AF pour des allèles hétérozygotes chez le premier parent et absents chez le second parent (sous-catégorie 3B) ;
- le calcul des valeurs AF moyennes, des valeurs AF moyennes ajustées ou des valeurs AF médianes des variants polymorphiques pour chacune des sous-catégories données, dans lequel les variants polymorphiques sont localisés entre deux localisations génomiques sur un chromosome et le fait d'évaluer si une anomalie génétique est présente dans le matériel génétique du sujet en fonction des valeurs AF des variants polymorphiques dans une ou plusieurs des sous-catégories et de la localisation génomique desdits variants polymorphiques ; et dans lequel une anomalie génétique est présente dans le matériel génétique du sujet lorsque les valeurs AF s'écartent de 0.5 ; en particulier lorsque la valeur AF s'écarte de 0.5 avec une valeur d'au moins et d'environ 0.025 ; plus particulièrement lorsque la valeur AF s'écarte de 0.5 avec une valeur d'au moins et d'environ 0.045.

**2.** Procédé selon la revendication 1, comprenant en outre ;

- le calcul de la différence entre les valeurs AF médianes, les valeurs AF moyennes ou les valeurs AF moyennes ajustées des sous-catégories 1A et 1B ou entre les valeurs AF médianes, les valeurs AF moyennes ou les valeurs AF moyennes ajustées des sous-catégories 2A et 2B, ou entre les valeurs AF médianes, les valeurs AF moyennes ou les valeurs AF moyennes ajustées des sous-catégories 3A et 3B ladite différence étant indiquée en tant que « delta AF » ;

- le fait d'évaluer si une anomalie génétique est présente dans le matériel génétique du sujet en fonction de valeurs « delta AF » observées entre lesdites localisations génomiques, et dans lequel une anomalie génétique est présente lorsque la valeur delta AF s'écarte de 0 ; en particulier lorsque la valeur delta AF s'écarte de 0 avec une valeur d'au moins et d'environ 0.05 ; plus particulièrement lorsque la valeur delta AF s'écarte de 0 avec une valeur d'au moins et d'environ 0.09.

3. Procédé selon les revendications 1 ou 2 dans lequel des variants polymorphiques ou des SNP qui sont distribués à moins de 50 kb, de préférence à moins de 20 kb les uns des autres sont retirés d'une analyse ultérieure.

4. Procédé selon l'une quelconque des revendications précédentes dans lequel les valeurs delta AF sont utilisées pour calculer une valeur pour la contribution parentale entre lesdites localisations génomiques et pour évaluer si une anomalie génétique est présente dans le matériel génétique du sujet en fonction de ladite valeur pour la contribution parentale observée entre lesdites localisations génomiques.

5. Procédé selon la revendication 4, dans lequel le calcul d'une valeur pour la contribution parentale entre lesdites localisations génomiques, et en particulier une valeur pour le pourcentage de contribution parentale (%Mat ou %Pat), est en fonction d'un modèle linéaire généralisé de second ordre entre les valeurs delta AF et le pourcentage de contribution parentale (%Mat ou % Pat) à travers lesdites localisations génomiques ; et dans lequel une contribution parentale s'écartant de 50 % ; en particulier un écart d'au moins et d'environ 3 % indique une anomalie chromosomique.

6. Procédé selon la revendication 4, dans lequel un %Mat ou %Pat entre et d'environ 44,4 % et 55,6 % indique une disomie normale ; dans lequel un %Mat ou %Pat entre et d'environ 63,6 % et 72,7 % indique une trisomie ; et dans lequel un %Mat ou %Pat entre et d'environ 0 % et 3,3 %, indique une monosomie.

7. Procédé selon l'une quelconque des revendications précédentes dans lequel l'allèle sélectionné par variant polymorphique est un allèle avec une caractéristique spécifique, ladite caractéristique étant sélectionnée parmi l'allèle A, l'allèle B, l'allèle avec la fréquence allélique la plus élevée dans une population donnée, l'allèle avec la fréquence allélique la plus faible dans une population donnée, un allèle de référence dans un génome de référence donné, l'allèle présent dans un état homozygote chez le parent 1, l'allèle présent dans un état homozygote chez le parent 2, l'allèle présent dans un état hétérozygote chez le parent 1, mais absent chez le parent 2, ou l'allèle présent dans un état hétérozygote chez le parent 2 mais absent chez le parent 1 ; de préférence dans lequel l'allèle sélectionné par variant polymorphique est l'allèle B ; même plus préférablement dans lequel l'allèle sélectionné par variant polymorphique est l'allèle B comprenant un polymorphisme mononucléotidique (SNP).

8. Procédé selon l'une quelconque des revendications précédentes dans lequel les valeurs AF sélectionnées des variants polymorphiques des sous-catégories 2A, 2B, 3A et/ou 3B sont converties en appels génotypiques discrets et dans lequel il est évalué si les valeurs de fréquence allélique homozygotes ou hétérozygotes sont sous-représentées ou sur-représentées entre deux localisations génomiques particulières.

9. Procédé selon l'une quelconque des revendications précédentes dans lequel le matériel génétique du sujet est isolé d'un échantillon comprenant une faible quantité de matériel génétique dudit sujet ; tel qu'un échantillon ne comprenant qu'une seule ou que quelques cellules dudit sujet ; ou un échantillon de plasma obtenu auprès d'une mère enceinte dudit sujet.

10. Procédé selon l'une quelconque des revendications précédentes, dans lequel l'anomalie génétique comprend une anomalie chromosomique numérique ou structurelle présente dans toutes (non mosaïque) ou dans seulement une partie des cellules biopsiées (mosaïque) ; en particulier une anomalie chromosomique numérique ou structurelle sélectionnée parmi une monosomie, une disomie uniparentale, une trisomie, une tétrasomie, une duplication, une délétion, respectivement une monosomie en mosaïque, une disomie en mosaïque, une trisomie en mosaïque, une tétrasomie en mosaïque, une duplication en tandem en mosaïque, une délétion en mosaïque et des combinaisons de celles-ci.

11. Procédé selon la revendication 10, dans lequel lorsque la valeur delta AF s'écarte de 0, en dessous du seuil d'environ 0.09 (en particulier en dessous de 0.0924), la valeur est considérée comme normale (disomie normale) ; lorsque la valeur delta AF s'écarte de 0, au-dessus d'un seuil d'environ 0.2 (en particulier au-dessus de 0.234) et qu'un nombre de copies accru est observé, la valeur indique une trisomie complète ou une duplication ; et lorsque la valeur delta AF s'écarte de 0. entre l'une et l'autre des valeurs seuils et qu'un nombre de copies accru est observé, l'échantillon est catégorisé en tant que trisomie/disomie en mosaïque ou duplication en mosaïque.

12. Procédé selon la revendication 10, dans lequel lorsque la valeur delta AF s'écarte de 0, en dessous du seuil d'environ 0.09 (en particulier en dessous de 0.0924), la valeur est considérée comme normale (disomie normale) ; lorsque la valeur delta AF s'écarte de 0 au-dessus d'un seuil d'environ 0.9 et qu'un nombre de copies diminué est observé, la valeur indique une monosomie ou délétion complète ; et lorsque la valeur delta AF s'écarte de 0, entre l'une et l'autre des valeurs seuils et qu'un nombre de copies diminué est observé, l'échantillon est catégorisé en tant que monosomie/disomie en mosaïque ou délétion en mosaïque.

13. Procédé selon l'une quelconque des revendications précédentes, dans lequel les variants polymorphiques sont sélectionnés parmi des polymorphismes mononucléotidiques (SNP), des séquences répétées en tandem courtes (STR) ; sélectionnées de préférence parmi des polymorphismes mononucléotidiques.

14. Produit programme d'ordinateur qui est capable, lorsqu'il est exécuté sur un moteur de traitement, de mettre en œuvre le procédé selon l'une quelconque des revendications précédentes.

15. Support de stockage non transitoire lisible par machine stockant le produit programme d'ordinateur selon la revendication 14.

Fig. 1

**Fig. 1 (continued)**

Fig. 1 (continued)

Fig. 2

A

**Fig. 2 (continued)**

EP 4 118 652 B1

Fig. 2 (continued)

C

Fig. 3

Fig. 4

Fig.5

**Fig. 7**

**Fig. 6**

Fig. 8

Fig. 8 (continued)

Fig. 9

Fig.9 (continued)

Fig.9 (continued)

## REFERENCES CITED IN THE DESCRIPTION

**Patent documents cited in the description**

- WO 2015028576 A

**Non-patent literature cited in the description**

- **DE RYCKE, M., A** ; **DE VOS, F. BELVA** ; **V. BERCKMOES** ; **M. BONDUELLE** ; **A. BUYSSE** ; **K. KEYMOLEN** ; **I. LIEBAERS** ; **J. NEKKEBROECK** ; **P. VERDYCK** ; **W. VERPOEST**. Preimplantation genetic testing with HLA matching: from counseling to birth and beyond. *J Hum Genet*, 2020
- **FRAGOULI, E.** ; **S. ALFARAWATI** ; **K. SPATH** ; **D. BABARIYA** ; **N. TAROZZI** ; **A. BORINI** ; **D. WELLS**. Analysis of implantation and ongoing pregnancy rates following the transfer of mosaic diploid-aneuploid blastocysts. *Hum Genet*, 2017, vol. 136, 805-819
- **GRECO, E.** ; **M. G. MINASI** ; **F. FIORENTINO**. Healthy Babies after Intrauterine Transfer of Mosaic Aneuploid Blastocysts. *N Engl J Med*, 2015, vol. 373, 2089-90
- **HANDYSIDE, A. H** ; **G. L. HARTON** ; **B. MARIANI** ; **A. R. THORNHILL** ; **N. AFFARA** ; **M. A. SHAW** ; **D. K. GRIFFIN**. Karyomapping: a universal method for genome wide analysis of genetic disease based on mapping crossovers between parental haplotypes. *J Med Genet*, 2010, vol. 47, 651-8
- **KUBICEK, D.** ; **M. HORNAK** ; **J. HORAK** ; **R. NAVRATIL** ; **G. TAUWINKLOVA** ; **J. RUBES & K. VESELA**. Incidence and origin of meiotic whole and segmental chromosomal aneuploidies detected by karyomapping. *Reprod Biomed Online*, 2019, vol. 38, 330-339
- **MUNNE, S.** ; **J. BLAZEK** ; **M. LARGE** ; **P. A. MARTINEZ-ORTIZ** ; **H. NISSON** ; **E. LIU** ; **N. TAROZZI** ; **A. BORINI** ; **A. BECKER** ; **J. ZHANG**. Detailed investigation into the cytogenetic constitution and pregnancy outcome of replacing mosaic blastocysts detected with the use of high-resolution next-generation sequencing. *Fertil Steril*, 2017, vol. 108, 62-71.e8
- **MUNNÉ, S** ; **F. SPINELLA** ; **J. GRIFO** ; **J. ZHANG** ; **M. P. BELTRAN** ; **E. FRAGOULI** ; **F. FIORENTINO**. Clinical outcomes after the transfer of blastocysts characterized as mosaic by high resolution Next Generation Sequencing- further insights. *Eur J Med Genet*, 2020, vol. 63, 103741
- **NATESAN, S. A** ; **A. H. HANDYSIDE** ; **A. R. THORNHILL** ; **C. S. OTTOLINI** ; **K. SAGE** ; **M. C. SUMMERS** ; **M. KONSTANTINIDIS** ; **D. WELLS** ; **D. K. GRIFFIN**. Live birth after PGD with confirmation by a comprehensive approach (karyomapping) for simultaneous detection of monogenic and chromosomal disorders. *Reprod Biomed Online*, 2014, vol. 29, 600-5
- **OTTOLINI, C. S** ; **S. ROGERS** ; **K. SAGE** ; **M. C. SUMMERS** ; **A. CAPALBO** ; **D. K. GRIFFIN** ; **J. SARASA** ; **D. WELLS** ; **A. H. HANDYSIDE**. Karyomapping identifies second polar body DNA persisting to the blastocyst stage: implications for embryo biopsy. *Reprod Biomed Online*, 2015, vol. 31, 776-82
- **SCHINZEL, A**. Catalogue of unbalanced chromosome aberrations in man. Walter de Gruyter, 2001
- **SPINELLA, F.** ; **F. FIORENTINO** ; **A. BIRICIK** ; **S. BONO** ; **A. RUBERTI** ; **E. COTRONEO** ; **M. BALDI** ; **E. CURSIO** ; **M. G. MINASI** ; **E. GRECO**. Extent of chromosomal mosaicism influences the clinical outcome of in vitro fertilization treatments. *Fertil Steril*, 2018, vol. 109, 77-83
- **VICTOR, A. R** ; **J. C. TYNDALL** ; **A. J. BRAKE** ; **L. T. LEPKOWSKY** ; **A. E. MURPHY** ; **D. K. GRIFFIN** ; **R. C. MCCOY** ; **F. L. BARNES** ; **C. G. ZOUVES** ; **M. VIOTTI**. One hundred mosaic embryos transferred prospectively in a single clinic: exploring when and why they result in healthy pregnancies. *Fertil Steril*, 2019, vol. 111, 280-293
- **ZAMANI ESTEKI, M** ; **E. DIMITRIADOU** ; **L. MATEIU** ; **C. MELOTTE, N** ; **VAN DER AA** ; **P. KUMAR** ; **R. DAS** ; **K. THEUNIS** ; **J. CHENG** ; **E. LEGIUS**. Concurrent whole-genome haplotyping and copy-number profiling of single cells. *Am J Hum Genet*, 2015, vol. 96, 894-912
- **ZHANG, L** ; **D. WEI** ; **Y. ZHU** ; **Y. GAO** ; **J. YAN** ; **Z. J. CHEN**. Rates of live birth after mosaic embryo transfer compared with euploid embryo transfer. *J Assist Reprod Genet*, 2019, vol. 36, 165-172

- **ZHANG, Y. X.** ; **J. J. CHEN** ; **S. NABU** ; **Q. S. Y. YEUNG** ; **Y. LI** ; **J. H. TAN** ; **W. SUKSALAK** ; **S. CHANCHAMROEN** ; **W. QUANGKANANURUG** ; **P. S. WONG**. The Pregnancy Outcome of Mosaic Embryo Transfer: A Prospective Multicenter Study and Meta-Analysis. *Genes (Basel)*, 2020, vol. 11

- **ZORE, T.** ; **L. L. KROENER** ; **C. WANG** ; **L. LIU** ; **R. BUYALOS** ; **G. HUBERT** ; **M. SHAMONKI**. Transfer of embryos with segmental mosaicism is associated with a significant reduction in live-birth rate. *Fertil Steril*, 2019, vol. 111, 69-76